# EUROPEAN PATENT SPECIFICATION

(11) **EP 4 243 785 B1**
(45) Date of publication and mention of the grant of the patent: **08.04.2026**
(21) Application number: 21819209.4
(22) Date of filing: 11.11.2021
(51) Int. Cl.: A61K 9/48, A61P 35/00, A61K 31/5025

(54) **TALAZOPARIB SOFT GELATIN CAPSULE DOSAGE FORM**
DARREICHUNGSFORM FÜR TALAZOPARIB-WEICHGELATINEKAPSEL
FORME GALÉNIQUE DE CAPSULE DE GÉLATINE MOLLE DE TALAZOPARIB

(30) Priority: 13.11.2020 US 202063113345 P; 05.11.2021 US 202163276554 P
(43) Date of publication of application: 20.09.2023
(73) Proprietor: Pfizer Inc., New York, NY 10001-2192 (US)
(72) Inventor: CARMODY, Alan Francis, Sandwich, Kent CT13 9NJ (GB); PAIRET, Lydie Claude Sylvie, Sandwich, Kent CT13 9NJ (GB)
(74) Representative: Pfizer
(86) International application number: PCT/IB2021/060462
(87) International publication number: WO 2022/101828

(56) References cited:
- WO-A1-2018/122168
- US-A1- 2015 044 285
- US-A1- 2015 366 814
- US-A1- 2019 054 087

## Description

### Field of the Invention

The present invention relates to a soft gelatin capsule dosage form of talazoparib, or a pharmaceutically acceptable salt thereof. In particular, the present invention relates to a soft gelatin capsule dosage form of talazoparib tosylate. The invention also relates to medical uses of the soft gelatin capsule dosage form of the present invention.

### Background

Poly (ADP-ribose) polymerase (PARP) engages in the naturally occurring process of DNA repair in a cell. PARP inhibition has been shown to be an effective therapeutic strategy against tumors associated with germline mutation in double-strand DNA repair genes by inducing synthetic lethality (Sonnenblick, A., et al., Nat. Rev. Clin. Oncol, 2015, 12(1), 27-4).

Talazoparib is a potent, orally available small molecule PARP inhibitor, which is cytotoxic to human cancer cell lines harboring gene mutations that compromise deoxyribonucleic acid (DNA) repair, an effect referred to as synthetic lethality, and by trapping PARP protein on DNA thereby preventing DNA repair, replication, and transcription.

The compound, talazoparib, which is "(8S,9R)-5-fluoro-8-(4-fluorophenyl)-9-(1-methyl-1*H*-1,2,4-triazol-5-yl)-8,9-dihydro-2*H*-pyrido[4,3,2-*de*]phthalazin-3(7*H*)-one" and "(8*S*,9*R*)-5-fluoro-8-(4-fluorophenyl)-9-(1-methyl-1*H*-1,2,4-triazol-5-yl)-2,7,8,9-tetrahydro-3*H*-pyrido[4,3,2-*de*]phthalazin-3-one" (also referred to as "PF-06944076", "MDV3800", and "BMN673") is a PARP inhibitor, having the structure,

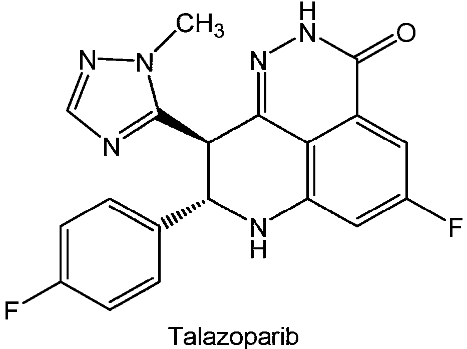

Talazoparib, and pharmaceutically acceptable salts thereof, including the tosylate salt, are disclosed in International Publication Nos. WO 2010/017055 and WO 2012/054698. Additional methods of preparing talazoparib, and pharmaceutically acceptable salts thereof, including the tosylate salt, are described in International Publication Nos. WO 2011/097602, WO 2015/069851, and WO 2016/019125. Additional methods of treating cancer using talazoparib, and pharmaceutically acceptable salts thereof, including the tosylate salt, are disclosed in International Publication Nos. WO 2011/097334 and WO 2017/075091. WO 2018/122168 A1 and US 2019/054087 A1 disclose compositions comprising talazoparib while US 2015/366814 A1 and US 2015/044285 A1 disclose some soft capsule compositions containing other active ingredients.

TALZENNA^{®} (talazoparib) (0.25 mg and 1 mg capsules) has been approved in several countries, including the United States, and in the European Union, and is approved or under review with anticipated approvals in other countries for the treatment of adult patients with deleterious or suspected deleterious gBRCAm HER2-negative locally advanced or metastatic breast cancer. Talazoparib is under development for a variety of human cancers both as a single agent and in combination with other agents. Additional capsule strengths, 0.5 mg and 0.75 mg, have been approved in the United States (see, for example, TALZENNA^{®} US Package Insert dated 9/2021).

The current commercial dosage form of talazoparib is an immediate release capsule (see, for example, TALZENNA^{®} US Package Insert dated 10/2020). The drug product consists of talazoparib tosylate drug substance formulated with succimidyl-4-N-maleimidomethyl cyclohexane-1-carboxylate (SMCC) filled into hydroxypropyl methylcellulose (HPMC) capsules. Generally, this dosage form is known in the art as a powder filled capsule. The recommended dose of TALZENNA^{®} is 1 mg administered orally once a day with or without food. Dosing interruptions or dose reductions of talazoparib to 0.75 mg, 0.5 mg or 0.25 mg once a day are allowed to manage adverse events with the use of the 0.25 mg commercial capsule. This dosage form, a powder filled capsule containing 0.25 mg or 1 mg of talazoparib, is considered in the pharmaceutical sciences to be a low dose and a low drug loading formulation. A "low dose" formulation means that there is a small amount of active drug in the formulation. "Drug loading", which is a known term in the pharmaceutical sciences art, is the ratio of the amount of active drug to the total content of the dosage form. Thus, a "low drug loading" formulation is a formulation that has a small amount of active drug compared to the total content of the dosage form.

The challenges in the manufacturing of low dose/low drug loading formulations are generally known within the pharmaceutical sciences (Gullapalli, R. P., J. Pharm. Sci., October 2010, 99(10), 4107-4148). Formulations that comprise simple binary mixtures of drug substance and excipient, such as the TALZENNA^{®} (talazoparib) 0.25 mg and 1 mg capsules are known to be susceptible to the following challenges: 1) nonuniform distribution of the drug substance throughout the powder mixture during various manufacturing processes; 2) segregation of the powder mixture during various manufacturing steps; and 3) loss of potency due to the drug substance becoming entrained to the manufacturing equipment during the various manufacturing steps.

Additionally, for low drug loading formulations, it is common to observe batch size dependence. As batch size increases, it becomes more challenging to maintain potency and content uniformity.

Accordingly, there is a need to develop an alternative dosage form for the talazoparib product, TALZENNA^{®}. It would be advantageous to provide a dosage form, which can deliver a low dose of talazoparib, or a pharmaceutically acceptable salt thereof, with suitable content uniformity and suitable physical and chemical stability properties. Further, it would be advantageous to provide a pharmaceutical dosage form that is bioequivalent to the first approved commercial dosage form of talazoparib, an immediate release capsule comprising talazoparib tosylate. It would also be advantageous to provide a pharmaceutical dosage form that has a suitable food effect on the pharmacokinetics, when administered with or without food. Additionally, it would be advantageous to provide a pharmaceutical dosage form that facilitates greater flexibility in batch size production. These, and other advantages of the present invention, are apparent from the description below.

### Summary

The scope of the present invention is defined by the appended claims. addition, each of the embodiments below describing the invention envisions within its scope the pharmaceutically acceptable salts of talazoparib.

The present invention relates to a pharmaceutical soft gelatin capsule dosage form comprising a soft gelatin shell and a fill, wherein:
(a) the soft gelatin shell comprises acid bone gelatin and at least one plasticizer; and
(b) the fill comprises an anti-oxidant, at least one solvent, and talazoparib or a pharmaceutically acceptable salt thereof.

One embodiment of the present invention relates to a pharmaceutical soft gelatin capsule dosage form, wherein the gelatin has a bloom strength of 175-200 Bloom.

One embodiment of the present invention relates to a pharmaceutical soft gelatin capsule dosage form, wherein the gelatin has a bloom strength of 195 Bloom.

One embodiment of the present invention relates to a pharmaceutical soft gelatin capsule dosage form, wherein the at least one plasticizer is a) glycerol; b) sorbitol; or c) glycerol and sorbitol.

One embodiment of the present invention relates to a pharmaceutical soft gelatin capsule dosage form, wherein the at least one plasticizer is one plasticizer or two plasticizers; the at least one plasticizer is one plasticizer; or the at least one plasticizer is two plasticizers.

One embodiment of the present invention relates to a pharmaceutical soft gelatin capsule dosage form, wherein the at least one plasticizer is one plasticizer which is glycerol or sorbitol; or the at least one plasticizer is two plasticizers which are glycerol and sorbitol.

One embodiment of the present invention relates to a pharmaceutical soft gelatin capsule dosage form, wherein the at least one plasticizer is one plasticizer which is glycerol or sorbitol; and further wherein the glycerol is glycerol 85%.

One embodiment of the present invention relates to a pharmaceutical soft gelatin capsule dosage form, wherein the at least one plasticizer is two plasticizers which are glycerol and sorbitol; and further wherein the glycerol is glycerol 85%.

One embodiment of the present invention relates to a pharmaceutical soft gelatin capsule dosage form, wherein the at least one plasticizer is one plasticizer which is glycerol or sorbitol; and further wherein the sorbitol is anhydrized liquid sorbitol NF.

One embodiment of the present invention relates to a pharmaceutical soft gelatin capsule dosage form, wherein the at least one plasticizer is two plasticizers which are glycerol and sorbitol; and further wherein the sorbitol is anhydrized liquid sorbitol NF.

One embodiment of the present invention relates to a pharmaceutical soft gelatin capsule dosage form, wherein the at least one plasticizer is one plasticizer which is glycerol or sorbitol; wherein the glycerol is glycerol 85%; and wherein the sorbitol is anhydrized liquid sorbitol NF.

One embodiment of the present invention relates to a pharmaceutical soft gelatin capsule dosage form, wherein the at least one plasticizer is two plasticizers which are glycerol and sorbitol; wherein the glycerol is glycerol 85%; and wherein the sorbitol is anhydrized liquid sorbitol NF.

One embodiment of the present invention relates to a pharmaceutical soft gelatin capsule dosage form, wherein the anti-oxidant is tocopherol.

One embodiment of the present invention relates to a pharmaceutical soft gelatin capsule dosage form, wherein the tocopherol is all-rac-alpha-tocopherol.

One embodiment of the present invention relates to a pharmaceutical soft gelatin capsule dosage form, wherein the at least one solvent is a) polyethylene glycol; b) glycerol; or c) polyethylene glycol and glycerol.

One embodiment of the present invention relates to a pharmaceutical soft gelatin capsule dosage form, wherein the at least one solvent is one solvent or two solvents; the at least one solvent is one solvent; and the at least one solvent is two solvents.

One embodiment of the present invention relates to a pharmaceutical soft gelatin capsule dosage form, wherein the at least one solvent is one solvent which is polyethylene glycol or glycerol; or the at least one solvent is two solvents which are polyethylene glycol and glycerol.

One embodiment of the present invention relates to a pharmaceutical soft gelatin capsule dosage form, wherein the at least one solvent is one solvent which is polyethylene glycol or glycerol; and further wherein the polyethylene glycol is polyethylene glycol 400.

One embodiment of the present invention relates to a pharmaceutical soft gelatin capsule dosage form, wherein the at least one solvent is two solvents which are polyethylene glycol and glycerol; and wherein the polyethylene glycol is polyethylene glycol 400.

One embodiment of the present invention relates to a pharmaceutical soft gelatin capsule dosage form, wherein the at least one solvent is one solvent which is polyethylene glycol or glycerol; and wherein the glycerol is glycerol 85%.

One embodiment of the present invention relates to a pharmaceutical soft gelatin capsule dosage form, wherein the at least one solvent is two solvents which are polyethylene glycol and glycerol; and wherein the glycerol is glycerol 85%.

One embodiment of the present invention relates to a pharmaceutical soft gelatin capsule dosage form, wherein the at least one solvent is one solvent which is polyethylene glycol or glycerol; wherein the polyethylene glycol is polyethylene glycol 400; and wherein the glycerol is glycerol 85%.

One embodiment of the present invention relates to a pharmaceutical soft gelatin capsule dosage form, wherein the at least one solvent is two solvents which are polyethylene glycol and glycerol; wherein the polyethylene glycol is polyethylene glycol 400; and wherein the glycerol is glycerol 85%.

One embodiment of the present invention relates to a pharmaceutical soft gelatin capsule dosage form, wherein the gelatin is acid bone gelatin and the anti-oxidant is tocopherol.

One embodiment of the present invention relates to a pharmaceutical soft gelatin capsule dosage form, wherein the gelatin is acid bone gelatin and the tocopherol is all-rac-alpha-tocopherol.

One embodiment of the present invention relates to a pharmaceutical soft gelatin capsule dosage form, wherein the gelatin is acid bone gelatin; the at least one plasticizer is one plasticizer which is glycerol or sorbitol; the anti-oxidant is tocopherol; and the at least one solvent is one solvent which is polyethylene glycol or glycerol.

One embodiment of the present invention relates to a pharmaceutical soft gelatin capsule dosage form, wherein the gelatin is acid bone gelatin; the at least one plasticizer is two plasticizers which are glycerol and sorbitol; the anti-oxidant is tocopherol; and the at least one solvent is two solvents which are polyethylene glycol and glycerol.

One embodiment of the present invention relates to a pharmaceutical soft gelatin capsule dosage form, wherein the gelatin is acid bone gelatin; the at least one plasticizer is one plasticizer which is glycerol or sorbitol; the tocopherol is all-rac-alpha-tocopherol; and the at least one solvent is one solvent which is polyethylene glycol or glycerol.

One embodiment of the present invention relates to a pharmaceutical soft gelatin capsule dosage form, wherein the gelatin is acid bone gelatin; the at least one plasticizer is two plasticizers which are glycerol and sorbitol; the tocopherol is all-rac-alpha-tocopherol; and the at least one solvent is two solvents which are polyethylene glycol and glycerol.

One embodiment of the present invention relates to a pharmaceutical soft gelatin capsule dosage form, wherein the dosage form comprises talazoparib, or a pharmaceutically acceptable salt thereof, in an amount equivalent to about 0.1 mg talazoparib free base.

One embodiment of the present invention relates to a pharmaceutical soft gelatin capsule dosage form, wherein the dosage form comprises talazoparib, or a pharmaceutically acceptable salt thereof, in an amount equivalent to about 0.25 mg talazoparib free base.

One embodiment of the present invention relates to a pharmaceutical soft gelatin capsule dosage form, wherein the dosage form comprises talazoparib, or a pharmaceutically acceptable salt thereof, in an amount equivalent to about 0.35 mg talazoparib free base.

One embodiment of the present invention relates to a pharmaceutical soft gelatin capsule dosage form, wherein the dosage form comprises talazoparib, or a pharmaceutically acceptable salt thereof, in an amount equivalent to about 0.5 mg talazoparib free base.

One embodiment of the present invention relates to a pharmaceutical soft gelatin capsule dosage form, wherein the dosage form comprises talazoparib, or a pharmaceutically acceptable salt thereof, in an amount equivalent to about 0.75 mg talazoparib free base.

One embodiment of the present invention relates to a pharmaceutical soft gelatin capsule dosage form, wherein the dosage form comprises talazoparib, or a pharmaceutically acceptable salt thereof, in an amount equivalent to about 1 mg talazoparib free base.

One embodiment of the present invention relates to a pharmaceutical soft gelatin capsule dosage form, wherein the talazoparib, or a pharmaceutically acceptable salt thereof, is talazoparib tosylate.

One embodiment of the present invention relates to a pharmaceutical soft gelatin capsule dosage form, wherein the dosage form is for oral administration.

The present invention also relates to a pharmaceutical soft gelatin capsule dosage form comprising a soft gelatin shell and a fill,
wherein the soft gelatin shell comprises:
   about 60 percent to about 61 percent by weight of acid bone gelatin,
   about 15 percent to about 16 percent by weight of glycerol, and
   about 23 percent of sorbitol; and
wherein, the fill comprises:
   about 0.3 percent by weight of tocopherol,
   about 95 percent by weight of polyethylene glycol,
   about 4 percent by weight of glycerol, and
   talazoparib, or a pharmaceutically acceptable salt thereof in about 0.15 percent to about 0.3 percent by weight free base equivalent.

One embodiment of the present invention relates to a pharmaceutical soft gelatin capsule dosage form, wherein the gelatin has a bloom strength of 175-200 Bloom.

One embodiment of the present invention relates to a pharmaceutical soft gelatin capsule dosage form, wherein the gelatin has a bloom strength of 195 Bloom.

One embodiment of the present invention relates to a pharmaceutical soft gelatin capsule dosage form, wherein the glycerol is glycerol 85%.

One embodiment of the present invention relates to a pharmaceutical soft gelatin capsule dosage form, wherein the sorbitol is anhydrized liquid sorbitol NF.

One embodiment of the present invention relates to a pharmaceutical soft gelatin capsule dosage form, wherein the tocopherol is all-rac-alpha-tocopherol.

One embodiment of the present invention relates to a pharmaceutical soft gelatin capsule dosage form, wherein the polyethylene glycol is polyethylene glycol 400.

One embodiment of the present invention relates to a pharmaceutical soft gelatin capsule dosage form, wherein the amount of talazoparib or a pharmaceutically acceptable salt thereof, is equivalent to about 0.1 mg talazoparib free base.

One embodiment of the present invention relates to a pharmaceutical soft gelatin capsule dosage form, wherein the amount of talazoparib or a pharmaceutically acceptable salt thereof, is equivalent to about 0.25 mg talazoparib free base.

One embodiment of the present invention relates to a pharmaceutical soft gelatin capsule dosage form, wherein the amount of talazoparib or a pharmaceutically acceptable salt thereof, is equivalent to about 0.35 mg talazoparib free base.

One embodiment of the present invention relates to a pharmaceutical soft gelatin capsule dosage form, wherein the amount of talazoparib or a pharmaceutically acceptable salt thereof, is equivalent to about 0.5 mg talazoparib free base.

One embodiment of the present invention relates to a pharmaceutical soft gelatin capsule dosage form, wherein the amount of talazoparib or a pharmaceutically acceptable salt thereof, is equivalent to about 0.75 mg talazoparib free base.

One embodiment of the present invention relates to a pharmaceutical soft gelatin capsule dosage form, wherein the amount of talazoparib or a pharmaceutically acceptable salt thereof, is equivalent to about 1 mg talazoparib free base.

The present invention relates to a pharmaceutical soft gelatin capsule dosage form comprising a soft gelatin shell and a fill,
wherein the soft gelatin shell comprises:
   about 46.3 mg/capsule of acid bone gelatin,
   about 11.8 mg/capsule of glycerol, and
   about 17.6 mg/capsule of sorbitol; and
wherein, the fill comprises:
   about 0.3 mg/capsule of tocopherol,
   about 95.6 mg/capsule of polyethylene glycol,
   about 4 mg/capsule of glycerol, and
   about 0.1 mg of talazoparib free base, or an equivalent amount of a pharmaceutically acceptable salt thereof.

The present invention relates to a pharmaceutical soft gelatin capsule dosage form comprising a soft gelatin shell and a fill,
wherein the soft gelatin shell comprises:
   about 46.270 mg/capsule of acid bone gelatin,
   about 11.790 mg/capsule of glycerol, and
   about 17.57 mg/capsule of sorbitol; and
wherein, the fill comprises:
   about 0.300 mg/capsule of tocopherol,
   about 95.555 mg/capsule of polyethylene glycol,
   about 4.000 mg/capsule of glycerol, and
   about 0.1 mg of talazoparib free base, or an equivalent amount of a pharmaceutically acceptable salt thereof.

The present invention relates to a pharmaceutical soft gelatin capsule dosage form comprising a soft gelatin shell and a fill,
wherein the soft gelatin shell comprises:
   about 61.1 mg/capsule of acid bone gelatin,
   about 16.6 mg/capsule of glycerol, and
   about 23.2 mg/capsule of sorbitol; and
wherein, the fill comprises:
   about 0.4 mg/capsule of tocopherol,
   about 119 mg/capsule of polyethylene glycol,
   about 5 mg/capsule of glycerol, and
   about 0.25 mg of talazoparib free base, or an equivalent amount of a pharmaceutically acceptable salt thereof.

The present invention relates to a pharmaceutical soft gelatin capsule dosage form comprising a soft gelatin shell and a fill,
wherein the soft gelatin shell comprises:
   about 61.097 mg/capsule of acid bone gelatin,
   about 16.610 mg/capsule of glycerol, and
   about 23.190 mg/capsule of sorbitol; and
wherein, the fill comprises:
   about 0.375 mg/capsule of tocopherol,
   about 119.262 mg/capsule of polyethylene glycol,
   about 5.000 mg/capsule of glycerol, and
   about 0.25 mg of talazoparib free base, or an equivalent amount of a pharmaceutically acceptable salt thereof.

The present invention relates to a pharmaceutical soft gelatin capsule dosage form comprising a soft gelatin shell and a fill,
wherein the soft gelatin shell comprises:
   about 78 mg/capsule of acid bone gelatin,
   about 20 mg/capsule of glycerol, and
   about 30 mg/capsule of sorbitol; and
wherein, the fill comprises:
   about 0.5 mg/capsule of tocopherol,
   about 167 mg/capsule of polyethylene glycol,
   about 7 mg/capsule of glycerol, and
   about 0.35 mg of talazoparib free base, or an equivalent amount of a pharmaceutically acceptable salt thereof.

The present invention relates to a pharmaceutical soft gelatin capsule dosage form comprising a soft gelatin shell and a fill,
wherein the soft gelatin shell comprises:
   about 78.130 mg/capsule of acid bone gelatin,
   about 20.250 mg/capsule of glycerol, and
   about 29.66 mg/capsule of sorbitol; and
wherein, the fill comprises:
   about 0.509 mg/capsule of tocopherol,
   about 166.966 mg/capsule of polyethylene glycol,
   about 7.000 mg/capsule of glycerol, and
   about 0.35 mg of talazoparib free base, or an equivalent amount of a pharmaceutically acceptable salt thereof.

The present invention relates to a pharmaceutical soft gelatin capsule dosage form comprising a soft gelatin shell and a fill,
wherein the soft gelatin shell comprises:
   about 105 mg/capsule of acid bone gelatin,
   about 27 mg/capsule of glycerol, and
   about 40 mg/capsule of sorbitol; and
wherein, the fill comprises:
   about 0.8 mg/capsule of tocopherol,
   about 239 mg/capsule of polyethylene glycol,
   about 10 mg/capsule of glycerol, and
   about 0.5 mg of talazoparib free base, or an equivalent amount of a pharmaceutically acceptable salt thereof.

The present invention relates to apharmaceutical soft gelatin capsule dosage form comprising a soft gelatin shell and a fill,
wherein the soft gelatin shell comprises:
   about 104.930 mg/capsule of acid bone gelatin,
   about 26.640 mg/capsule of glycerol, and
   about 39.830 mg/capsule of sorbitol; and
wherein, the fill comprises:
   about 00.750 mg/capsule of tocopherol,
   about 238.523 mg/capsule of polyethylene glycol,
   about 10.000 mg/capsule of glycerol, and
   about 0.5 mg of talazoparib free base, or an equivalent amount of a pharmaceutically acceptable salt thereof.

The present invention relates to apharmaceutical soft gelatin capsule dosage form comprising a soft gelatin shell and a fill,
wherein the soft gelatin shell comprises:
   about 141 mg/capsule of acid bone gelatin,
   about 36 mg/capsule of glycerol, and
   about 54 mg/capsule of sorbitol; and
wherein, the fill comprises:
   about 1 mg/capsule of tocopherol,
   about 358 mg/capsule of polyethylene glycol,
   about 15 mg/capsule of glycerol, and
   about 0.75 mg of talazoparib free base, or an equivalent amount of a pharmaceutically acceptable salt thereof.

The present invention relates to a pharmaceutical soft gelatin capsule dosage form comprising a soft gelatin shell and a fill,
wherein the soft gelatin shell comprises:
   about 141.260 mg/capsule of acid bone gelatin,
   about 36.000 mg/capsule of glycerol, and
   about 53.620 mg/capsule of sorbitol; and
wherein, the fill comprises:
   about 1.125 mg/capsule of tocopherol,
   about 357.784 mg/capsule of polyethylene glycol,
   about 15.000 mg/capsule of glycerol, and
   about 0.75 mg of talazoparib free base, or an equivalent amount of a pharmaceutically acceptable salt thereof.

The present invention relates to a pharmaceutical soft gelatin capsule dosage form comprising a soft gelatin shell and a fill,
wherein the soft gelatin shell comprises:
   about 178 mg/capsule of acid bone gelatin,
   about 45 mg/capsule of glycerol, and
   about 67 mg/capsule of sorbitol; and

wherein, the fill comprises;
   about 1.5 mg/capsule of tocopherol,
   about 477 mg/capsule of polyethylene glycol,
   about 20 mg/capsule of glycerol, and
   about 1 mg of talazoparib free base, or an equivalent amount of a pharmaceutically acceptable salt thereof.

The present invention relates to a pharmaceutical soft gelatin capsule dosage form comprising a soft gelatin shell and a fill,
wherein the soft gelatin shell comprises:
   about 177.790 mg/capsule of acid bone gelatin,
   about 45.310 mg/capsule of glycerol, and
   about 67.490 mg/capsule of sorbitol; and
wherein, the fill comprises;
   about 1.453 mg/capsule of tocopherol,
   about 477.047mg/capsule of polyethylene glycol,
   about 20.000 mg/capsule of glycerol, and
   about 1 mg of talazoparib free base, or an equivalent amount of a pharmaceutically acceptable salt thereof.

One embodiment of the present invention relates to a pharmaceutical soft gelatin capsule dosage form, wherein the gelatin has a bloom strength of 175-200 Bloom.

One embodiment of the present invention relates to a pharmaceutical soft gelatin capsule dosage form, wherein the gelatin has a bloom strength of 195 Bloom.

One embodiment of the present invention relates to a pharmaceutical soft gelatin capsule dosage form, wherein the glycerol is glycerol 85%.

One embodiment of the present invention relates to a pharmaceutical soft gelatin capsule dosage form, wherein the sorbitol is anhydrized liquid sorbitol NF.

One embodiment of the present invention relates to a pharmaceutical soft gelatin capsule dosage form, wherein the tocopherol is all-rac-alpha-tocopherol.

One embodiment of the present invention relates to a pharmaceutical soft gelatin capsule dosage form, wherein the polyethylene glycol is polyethylene glycol 400.

One embodiment of the present invention relates to a pharmaceutical soft gelatin capsule dosage form, wherein the talazoparib, or a pharmaceutically acceptable salt thereof, is talazoparib tosylate.

One embodiment of the present invention relates to a pharmaceutical soft gelatin capsule dosage form, wherein the talazoparib, or a pharmaceutically acceptable salt thereof, is talazoparib free base.

One embodiment of the present invention relates to a pharmaceutical soft gelatin capsule dosage form, wherein the dosage form is for oral administration.

This invention relates to a pharmaceutical soft gelatin capsule dosage form according to any of the previous embodiments, wherein the dosage form provides a 90% confidence interval for a geometric mean for log-transformed AUC₂₄ within the range of 80% to 125% of the geometric mean for log-transformed AUC₂₄ for a powder filled immediate release oral capsule containing an equivalent amount of talazoparib after oral administration to a subject, wherein the subject is in a fasted condition.

This invention relates to a pharmaceutical soft gelatin capsule dosage according to any of the previous embodiments, wherein the dosage form provides a 90% confidence interval for a geometric mean for log-transformed Cₘₐₓ within the range of 80% to 125% of the geometric mean for log-transformed Cₘₐₓ for a powder filled oral capsule containing an equivalent amount of talazoparib after oral administration to a subject, wherein the subject is in a fasted condition.

This invention relates to a pharmaceutical soft gelatin capsule dosage form according to any of the previous embodiments, wherein the dosage form (a) provides a geometric mean for log-transformed AUC₂₄ in the range of 80% to 125% of the geometric mean for log-transformed AUC₂₄ for a powder filled oral capsule containing an equivalent amount of talazoparib after administration to a subject, wherein the subject is in a fasted condition; (b) provides a geometric mean for log-transformed Cₘₐₓ in the range of 80% to 125% of the geometric mean for log-transformed Cₘₐₓ for a powder filled oral capsule containing an equivalent amount of talazoparib after administration to a subject, wherein the subject is in a fasted condition; or (c) both (a) and (b).

This invention relates to a pharmaceutical soft gelatin capsule dosage form according to any of the previous embodiments, wherein the dosage form (a) provides a geometric mean for log-transformed AUC₂₄ in the range of 80% to 125% of the geometric mean for log-transformed AUC₂₄ for a powder filled oral capsule containing an equivalent amount of talazoparib after administration to a subject, wherein the subject is in a fasted condition.

This invention relates to a pharmaceutical soft gelatin capsule dosage form according to any of the previous embodiments, wherein the dosage form (a) has a geometric mean fed/fasted ratio for log-transformed AUC₂₄ from about 0.8 to about 1.25 after oral administration to a subject; (b) has a geometric mean fed/fasted ratio for log-transformed Cₘₐₓ from about 0.8 to about 1.25 after oral administration to a subject; or (c) both (a) and (b).

This invention relates to a pharmaceutical soft gelatin capsule dosage form according to any of the previous embodiments, wherein the dosage form (a) has a geometric mean fed/fasted ratio for log-transformed AUC₂₄ from about 0.8 to about 1.25 after oral administration to a subject.

This invention relates to a medical use of treating cancer in a subject comprising administering to the subject a pharmaceutical soft gelatin capsule dosage form of any of the previous embodiments.

One embodiment of the present invention relates a medical use of treating cancer in a subject comprising administering to the subject a pharmaceutical soft gelatin capsule dosage form, wherein the cancer is selected from the group consisting of non-small cell lung cancer, small cell lung cancer, breast cancer, ovarian cancer, and prostate cancer.

One embodiment of the present invention relates a medical use of treating cancer in a subject comprising administering to the subject a pharmaceutical soft gelatin capsule dosage form, wherein the cancer is breast cancer, and wherein the breast cancer is triple negative breast cancer, hormone positive breast cancer, and HER2 negative breast cancer.

One embodiment of the present invention relates a medical use of treating cancer in a subject comprising administering to the subject a pharmaceutical soft gelatin capsule dosage form, wherein the cancer is prostate cancer, and wherein the prostate cancer is castration-sensitive prostate cancer or castration-resistant prostate cancer.

One embodiment of the present invention relates a medical use of treating cancer in a subject comprising administering to the subject a pharmaceutical soft gelatin capsule dosage form, wherein the subject is a human.

### Brief Description of the Drawings

FIGURE 1 shows the flow diagram of the manufacturing process for the gelatin mass of talazoparib soft gelatin capsules.
FIGURE 2 shows the flow diagram of the manufacturing process for the fill and encapsulation of talazoparib tosylate soft gelatin capsules.
FIGURE 3 shows the formation of the degradation product, cis talazoparib, in talazoparib 0.1 mg soft gelatin capsules at 40°C/75 % relative humidity.
FIGURE 4 shows the formation of the degradation product, cis talazoparib, in talazoparib 0.1 mg soft gelatin capsules at 30°C/75 % relative humidity.
FIGURE 5 shows the formation of the degradation product, cis talazoparib, in talazoparib 1 mg soft gelatin capsules at 40°C/75 % relative humidity.
FIGURE 6 shows the formation of the degradation product, cis talazoparib, in talazoparib 1 mg soft gelatin capsules at 30°C/75 % relative humidity.

### Detailed Description

The present invention may be understood more readily by reference to the following detailed description of the preferred embodiments of the invention. It is to be understood that the terminology used herein is for the purpose of describing specific embodiments. It is further to be understood that unless specifically defined herein, the terminology used herein is to be given its traditional meaning as known in the relevant art.

As used herein, the singular form "a", "an", and "the" include plural references unless indicated otherwise. For example, "a" plasticizer includes one or more plasticizers.

The term "about" when used to modify a numerically defined parameter (e.g., the amount of gelatin, the amount of anti-oxidant, etc.) means that the parameter may vary by as much as 10% above or below (± 10%) the stated numerical value for that parameter. For example, a component of a formulation which is about 4.0 mg should be understood to mean that the component may vary between 3.6 mg and 4.4 mg.

The abbreviation "w/wʺʺ means the amount by weight of a substance dissolved in a known amount (by weight) of liquid. The terms "percent by weight" and "weight percent" and the abbreviations "% w/w", "percent w/w", "wt%" are interchangeable and express as a percentage the number of grams of one ingredient in 100 g of solution. As a mathematical expression, the weight percent or the percent of solute in a solution is equal to the weight of solute/weight of solvent*100.

As used herein "pharmaceutically acceptable" means the component is suitable for oral administration to patients.

The term "pharmaceutically acceptable salt", as used herein, unless otherwise indicated, refers to a formulation of a compound that does not cause significant irritation to an organism to which it is administered and does not abrogate the biological activity and properties of the compound. In certain instances, pharmaceutically acceptable salts are obtained by reacting a compound described herein, with acids such as hydrochloric acid, hydrobromic acid, sulfuric acid, nitric acid, phosphoric acid, methanesulfonic acid, ethanesulfonic acid, p-toluenesulfonic acid, salicylic acid and the like. In some instances, pharmaceutically acceptable salts are obtained by reacting a compound having acidic group described herein with a base to form a salt such as an ammonium salt, an alkali metal salt, such as a sodium or a potassium salt, an alkaline earth metal salt, such as a calcium or a magnesium salt, a salt of organic bases such as dicyclohexylamine, *N*-methyl-D-glucamine, tris(hydroxymethyl)methylamine, and salts with amino acids such as arginine, lysine, and the like, or by other methods previously determined.

The term "glycerol 85%", as used herein, is a mixture of glycerol and water. Glycerol, such as glycerol 85%, is used as a solvent of the drug substance in the fill solution and as a direct plasticizer for the gelatin shell. Glycerol, such as glycerol 85%, interacts with the gelatin, forming a stable thermo reversible gel network by reducing the glass transition temperature of the gelatin.

As used herein, the term "anhydrized liquid sorbitol NF", which may also be referred to as "dry substance of sorbitol liquid, partially dehydrated", is a form of sorbitol which is frequently used in soft gelatin capsules. Sorbitol, such as anhydrized liquid sorbitol NF, acts as an indirect plasticizer by retaining water within the gelatin shell, therefore reducing the formation of crystalline structures, which make the gelatin shell hard and brittle.

The term "polyethylene glycol 400" or "PEG400", as used herein, is a commonly used fill material in soft gelatin capsules. For purposes of the present invention, the drug substance dissolves in polyethylene glycol 400, while still maintaining acceptable chemical stability.

The present invention is directed to a pharmaceutical soft gelatin capsule dosage form comprising a soft gelatin shell and a fill, wherein:
(a) the soft gelatin shell comprises acid bone gelatin and at least one plasticizer: and
(b) the fill comprises an anti-oxidant, at least one solvent, and talazoparib or a pharmaceutically acceptable salt thereof.

Soft gelatin capsules are well known and are often described as softgels. Soft gelatin capsules are a single unit solid dosage form or formulation comprising a liquid or semisolid fill encased by a one piece hermetically sealed elastic gelatin-based outer shell. The capsules are filled and sealed in one continuous process. The fill is encapsulated into the gelatin shell. The soft gelatin shell may be referred to as a gelatin mass before encapsulation. The fill may be a solution, a suspension, or a semisolid, and may be referred to as the fill formulation, fill material, capsule fill or fill. The fill includes the drug substance, which is dissolved in a suitable solvent or dispersed in a suitable diluent, producing a homogenous solution (i.e., liquid fill) or [homogenous] suspension (i.e., semisolid fill), respectively. In an embodiment of the present invention, the fill is a liquid.

Gelatin, such as gelatin in soft gelatin capsules, is categorized by its strength. The term "Bloom", as used herein, is a test to measure the strength of a gelatin. The test determines the weight in grams needed by a specified plunger (normally with a diameter of 12.7 mm (0.5 inch)) to depress the surface of the gel by 4 mm without breaking it at a specified temperature. The number of grams is called the Bloom value, and most gelatins are between 30 g Bloom (the weakest) and 300 g Bloom (the strongest). The higher a Bloom value, the higher the melting and gelling points of a gelatin, and the shorter the gelling times. Low bloom gelatin typically falls between the range of 50-125 Bloom. Medium bloom gelatin typically falls between the range of 175-225 Bloom. High bloom gelatin typically falls between the range of 225-325 Bloom. The gelatin bloom strength in a soft gelatin capsule of the present invention is typically about 150 to about 200 Bloom (or grams). In an embodiment of the present invention, the gelatin bloom strength in the soft gelatin capsule is between 175-225 Bloom. In an embodiment of the present invention, the gelatin bloom strength in the soft gelatin capsule is 175-200 Bloom. In an embodiment of the present invention, the gelatin bloom strength in the soft gelatin capsule is 195 Bloom. Exemplary manufacturers of softgels include Catalent Phama Solutions, Somerset, N.J., Pharmagel Engineering spa. Lodi, Italy and Soft Gel Technologies Inc., Commerce, California.

The soft gelatin capsule of the invention is a pharmaceutical dosage form or formulation that comprises a gelatin-based shell and a fill. In an embodiment, the soft gelatin capsule is a liquid-filled soft gelatin capsule dosage form or formulation.

In the present invention, gelatin is a component in the shell of the soft gelatin capsule. The soft gelatin shell comprises gelatin and a plasticizer. The shell may optionally include an opacifier and/or dyes. Gelatin is obtained by the partial hydrolysis of collagen derived from the skin, white connective tissue and bones of animals including cattle, pigs and fish. It mainly consists of water-soluble proteins (4-90% w/w) along with mineral salts (1-2% w/w) and water (8-15% w/w). The protein fraction contains amino acids linked by amide bonds in a polypeptide chain.

Collagen is a fibrous protein and the main constituent of animal skin, bone and connective tissue. It consists of a triple helix of three polypeptide chains with a molecular weight of approximately 300,000 Da. Denaturation involves breaking of the hydrogen bonds to destabilize the collagen helix resulting in a marked decrease in the molecular weight and the intrinsic viscosity. Hydrolysis of collagen by boiling bones or skins in water results in a low yield of impure gelatin with poor physical properties. Therefore, commercial manufacture of gelatin involves initial removal of contaminants before thermal denaturing with the aid of either a dilute acid to result in Type A gelatin or a dilute alkali to result in Type B gelatin. Gelatin is amphoteric in nature with its isoelectric points ranging from 6.0 to 9.0 for Type A gelatin and from 4.7 to 5.3 for Type B gelatin. It Is believed that the alkaline hydrolysis causes a greater degree of deamidation of the asparagine and glutamine amino acids in collagen, resulting in a larger number of free carboxylic acid compared to acid hydrolysis. Examples of suitable Type A gelatin include acid bone and acid hide gelatin. Examples of suitable Type B gelatin include limed bone gelatin. In the present invention, the gelatin is acid bone gelatin. In a preferred embodiment, the gelatin is acid bone gelatin 195 Bloom.

The soft gelatin capsule will generally contain water in an amount of about 1% to about 20%, more preferably about 3% to about 18%, still more preferably about 5% to about 14% by weight of the gelatin shell after the fill has been encapsulated and water has migrated from the capsule to the fill. Without being bound by theory. It is believed that the water in the gelatin capsule, e.g., 20% to 50% by weight, prior to filling, migrates at least in part to assist with gelling the fill and increasing its viscosity.

In an embodiment, gelatin is present in an amount of about 50% to about 75%, preferably about 55% to about 65%, and more preferably about 59% to about 61%, and even more preferably about 60% to about 61% by weight of the soft gelatin shell.

Any pharmaceutically acceptable plasticizer may be included as a component in the shell of the soft gelatin capsule. At least one plasticizer is included. In an embodiment, one or two plasticizers are included. In an embodiment, one plasticizer is included. In a preferred embodiment, two plasticizers are included. Non-limiting examples of suitable plasticizer include polyhydric alcohols such as sorbitol, glycerin, mannitol, xylitol, and sorbitan; dialkylphthalates; lower alkyl citrates wherein the lower alkyl has 1-6 carbon atoms; glycols and polyglycols including polyethylene glycols with a molecular weight range of about 200 to about 2,000, methoxyl-propylene-glycol, and 1,2-propylene glycol; esters of polyhydroxy-alcohols such as mono-, di-, and tri-acetate of glycerol, ricinoleic acid and esters thereof; and mixtures of the above. In an embodiment, the plasticizer comprises glycerol and sorbitol. In an embodiment, the plasticizers are glycerol and sorbitol, the glycerol is glycerol 85% and the sorbitol is anhydrized liquid sorbitol NF.

In an embodiment, total plasticizer is present in an amount of about 20% to about 55%, more preferably about 30% to about 45%, still more preferably about 35% to about 40% by weight of the soft gelatin shell.

In the present invention, the fill comprises an anti-oxidant, at least one solvent, and talazoparib or a pharmaceutically acceptable salt thereof. In one embodiment, the talazoparib, or a pharmaceutically acceptable salt, in the fill of the soft gel capsule is talazoparib tosylate. The fill contains ingredients in an amount that would be pharmaceutically acceptable for oral administration.

In the present invention, the fill comprises an anti-oxidant. Non-limiting examples of suitable antioxidants include tocopherol, tocopherol polyethylene glycol succinate, butylated hydroxytoluene, butylated hydroxyanisole, dodecyl gallate, octyl gallate, propyl gallate, ascorbyl palmitate, sodium ascorbate, and thymol. In an embodiment, the antioxidant is tocopherol, tocopherol polyethylene glycol succinate, butylated hydroxytoluene, or propyl gallate. In a preferred embodiment, the antioxidant is tocopherol. In a more preferred embodiment, the antioxidant is all-rac-alpha-tocopherol.

In an embodiment, anti-oxidant is present in an amount of about 0.05% to about 1% and more preferably about 0.1% to about 0.5% by weight of the fill.

In the present invention, the fill comprises at least one solvent. In an embodiment, one or two solvents are included. In an embodiment, one solvent is included. In a preferred embodiment, two solvents are included. Non-limiting examples of suitable solvents include propylene glycol, acetone, ethanol, butylene glycol, diethylene glycol monoethyl ether, dipropylene glycol, glycerin, glycerol, polyethylene glycol, mineral oil, peanut oil, corn oil, and sesame oil. In an embodiment, the solvent is glycerol. In an embodiment, the solvent is glycerol and the glycerol is glycerol 85%. In an embodiment, the solvent is polyethylene glycol. In an embodiment, the polyethylene glycol has a molecular weight range of about 200 to about 900. In an embodiment, the polyethylene glycol has a molecular weight less than 900. In an embodiment, the polyethylene glycol is polyethylene glycol 600 (PEG 600). In an embodiment, the polyethylene glycol is polyethylene glycol 400 (PEG 400). In an embodiment, the solvent comprises polyethylene glycol and glycerol. In a preferred embodiment, the solvents are polyethylene glycol and glycerol. In a more preferred embodiment, the solvents are polyethylene glycol and glycerol, the polyethylene glycol is PEG 400 and the glycerol is glycerol 85%.

In an embodiment of the present invention, wherein the solvent comprises polyethylene glycol and glycerol, the polyethylene glycol is present in the fill in the amount of about 80% to about 99%, preferably about 94% to about 98%, and more preferably of about 95% to about 96% by weight of the total weight of the fill; and the glycerol is present in the fill in an amount of about 1% to about 10%, preferably about 2% to about 6%, and more preferably about 4% by weight of the total weight of the fill.

In the present invention, talazoparib, or a pharmaceutically acceptable salt thereof, is a component in the fill of the soft gelatin capsule. In an embodiment of the present invention, the dosage form comprises talazoparib, or a pharmaceutically acceptable salt thereof, and preferably a tosylate thereof, in an amount equivalent to about 0.1 mg to about 1 mg talazoparib free base. In an embodiment of the present invention, the dosage form comprises talazoparib, or a pharmaceutically acceptable salt thereof, and preferably a tosylate thereof, in an amount equivalent to about 0.1 mg talazoparib free base; to about 0.25 mg talazoparib free base; to about 0.35 mg talazoparib free base; to about 0.5 mg talazoparib free base; to about 0.75 mg talazoparib free base; or to about 1 mg talazoparib free base.

In an embodiment of the present invention, the amount of talazoparib, or a pharmaceutically acceptable salt thereof, and preferably a tosylate thereof, is equivalent to about 0.1 mg to about 1 mg talazoparib free base. In an embodiment of the present invention, the amount of talazoparib, or a pharmaceutically acceptable salt thereof, and preferably a tosylate thereof, is equivalent to about 0.1 mg talazoparib free base; to about 0.25 mg talazoparib free base; to about 0.35 mg talazoparib free base; to about 0.5 mg talazoparib free base; to about 0.75 mg talazoparib free base; or to about 1 mg talazoparib free base.

In an embodiment of the present invention, the amount of talazoparib, or a pharmaceutically acceptable salt thereof, and preferably a tosylate thereof, is about 0.1 mg to about 1 mg talazoparib free base equivalent. In an embodiment of the present invention, the amount of talazoparib, or a pharmaceutically acceptable salt thereof, and preferably a tosylate thereof, is about 0.1 mg talazoparib free base equivalent; about 0.25 mg talazoparib free base equivalent; about 0.35 mg talazoparib free base equivalent; about 0.5 mg talazoparib free base equivalent; about 0.75 mg talazoparib free base equivalent; or about 1 mg talazoparib free base equivalent.

### Therapeutic Methods and Uses

"Abnormal cell growth", as used herein, unless otherwise indicated, refers to cell growth that is independent of normal regulatory mechanisms (e.g., loss of contact inhibition). Abnormal cell growth may be benign (not cancerous), or malignant (cancerous).

The terms "cancer", "cancerous", and "malignant" refer to or describe the physiological condition in mammals that is typically characterized by unregulated cell growth. As used herein "cancer" refers to any malignant and/or invasive growth or tumor caused by abnormal cell growth. As used herein "cancer" refers to solid tumors named for the type of cells that form them, cancer of blood, bone marrow, or the lymphatic system. Examples of solid tumors include but not limited to sarcomas and carcinomas. Examples of cancers of the blood include but not limited to leukemias, lymphomas and myeloma. The term "cancer" includes but is not limited to a primary cancer that originates at a specific site in the body, a metastatic cancer that has spread from the place in which it started to other parts of the body, a recurrence from the original primary cancer after remission, and a second primary cancer that is a new primary cancer in a person with a history of previous cancer of different type from latter one. Examples of cancer include, but are not limited to, carcinoma, lymphoma, leukaemia, blastoma, and sarcoma. More particular examples of such cancers include squamous cell carcinoma, myeloma, lung cancer, small-cell lung cancer, small cell prostate cancer, non-small cell lung cancer, glioma, Hodgkin's lymphoma, non-hogkin's lymphoma, follicular lymphoma (FL), diffuse large B-cell lymphoma (DLCBCL), acute myeloid leukaemia (AML), multiple myeloma, gastrointestinal (tract) cancer, renal cancer, ovarian cancer, uterine cancer, endometrial cancer, liver cancer, kidney cancer, renal cell carcinoma, prostate cancer, castration-sensitive prostate cancer, castration-resistant prostate cancer (CRPC), thyroid cancer, melanoma, chondrosarcoma, neuroblastoma, pancreatic cancer, glioblasoma, multiformer, cervical cancer, rectal cancer, brain cancer, stomach cancer, bladder cancer, hepatoma, hepatocellular carcinoma, breast cancer, colon cancer, head and neck cancer, and salivary gland cancer.

The term "patient" or "subject" refers to any single subject for which therapy is desired or that is participating in a clinical trial, epidemiological study or used as a control, including humans and mammalian veterinary patients such as cattle, horses, dogs and cats. In certain preferred embodiments, the subject is a human.

The term "treat" or "treating" a cancer as used herein means to administer a therapy according to the present invention to a subject having cancer, or diagnosed with cancer, to achieve at least one positive therapeutic effect, such as, for example, reduced number of cancer cells, reduced tumor size, reduced rate of cancer cell infiltration into peripheral organs, or reduced rate of tumor metastases or tumor growth, reversing, alleviating, inhibiting the progress of, or preventing the disorder or condition to which such term applies, or one or more symptoms of such disorder or condition. The term "treatment", as used herein, unless otherwise indicated, refers to the act of treating as "treating" is defined immediately above. The term "treating" also includes adjuvant and neo-adjuvant treatment of a subject. For the purposes of this invention, beneficial or desired clinical results include, but are not limited to, one or more of the following: reducing the proliferation of (or destroying) neoplastic or cancerous cells; inhibiting metastasis or neoplastic cells; shrinking or decreasing the size of tumor; remission of the cancer; decreasing symptoms resulting from the cancer; increasing the quality of life of those suffering from the cancer; decreasing the dose of other medications required to treat the cancer; delaying the progression the cancer; curing the cancer; overcoming one or more resistance mechanisms of the cancer; and / or prolonging survival of patients the cancer. Positive therapeutic effects in cancer can be measured in a number of ways (see, for example, W. A. Weber, J. Nucl. Med. 50:1S-10S (200)). In some embodiments, the treatment achieved by a method of the invention is any of the partial response (PR), complete response (CR), overall response (OR), objective response rate (ORR), progression free survival (PFS), radiographic PFS, disease free survival (DFS) and overall survival (OS). PFS, also referred to as "Time to Tumor Progression" indicates the length of time during and after treatment that the cancer does not grow, and includes the amount of time patients have experience a CR or PR, as well as the amount of time patients have experience stable disease (SD). DFS refers to the length of time during and after treatment that the patient remains free of disease. OS refers to a prolongation in life expectancy as compared to naïve or untreated subjects or patients. In some embodiments, response to a method of the invention is any of PR, CR, PFS, DFS, ORR, OR or OS. Response to a method of the invention, including duration of soft tissue response, is assessed using Response Evaluation Criteria in Solid Tumors version 1.1 (RECIST 1.1) response criteria. In some embodiments, the treatment achieved by a method of the invention is measured by the time to PSA progression, the time to initiation of cytotoxic chemotherapy and the proportion of patients with PSA response greater than or equal to 50%. The treatment regimen for a method of the invention that is effective to treat a cancer patient may vary according to factors such as the disease state, age, and weight of the patient, and the ability of the therapy to elicit an anti-cancer response in the subject. While an embodiment of any of the aspects of the invention may not be effective in achieving a positive therapeutic effect in every subject, it should do so in a statistically significant number of subjects as determined by any statistical test known in the art such as, but not limited to, the Cox log-rank test, the Cochran-Mantel-Haenszel log-rank test, the Student's t-test, the chi2-test, the U-test according to Mann and Whitney, the Kruskal-Wallis test (H-test), Jonckheere-Terpstrat-test and the Wilcon on-test. The term "treatment" also encompasses *in vitro* and *ex vivo* treatment, e.g., of a cell, by a reagent, diagnostic, binding compound, or by another cell.

As used herein, an "effective dosage" or "effective amount" of drug, compound or pharmaceutical formulation is an amount sufficient to effect any one or more beneficial or desired, including biochemical, histological and / or behavioral symptoms, of the disease, its complications and intermediate pathological phenotypes presenting during development of the disease. For therapeutic use, a "therapeutically effective amount" refers to that amount of a compound being administered which will relieve to some extent one or more of the symptoms of the disorder being treated. In reference to the treatment of cancer, a therapeutically effective amount refers to that amount which has the effect of (1) reducing the size of the tumor, (2) inhibiting (that is, slowing to some extent, preferably stopping) tumor metastasis, (3) inhibiting to some extent (that is, slowing to some extent, preferably stopping) tumor growth or tumor invasiveness, (4) relieving to some extent (or, preferably, eliminating) one or more signs or symptoms associated with the cancer, (5) decreasing the dose of other medications required to treat the disease, and / or (6) enhancing the effect of another medication, and / or delaying the progression of the disease of patients. An effective dosage can be administered in one or more administrations. For the purposes of this invention, an effective dosage of drug, compound, or pharmaceutical formulation is an amount sufficient to accomplish prophylactic or therapeutic treatment either directly or indirectly. As is understood in the clinical context, an effective dosage of drug, compound or pharmaceutical formulation may or may not be achieved in conjunction with another drug, compound or pharmaceutical formulation.

In an embodiment, an effective dosage of talazoparib, or a pharmaceutically acceptable salt thereof, and preferably a tosylate thereof, is administered at a daily dosage of from about 0.1 mg to about 2 mg once a day, preferably from about 0.25 mg to about 1.5 mg once a day, and more preferably from about 0.5 mg to about 1.0 mg once a day. In an embodiment, talazoparib or a pharmaceutically acceptable salt thereof, and preferably a tosylate thereof, is administered at a daily dosage of about 0.1 mg, about 0.25 mg, about 0.35 mg, about 0.5 mg, about 0.75 mg or about 1.0 mg once daily. In an embodiment, talazoparib or a pharmaceutically acceptable salt thereof, and preferably a tosylate thereof, is administered at a daily dosage of about 0.1 mg, about 0.25 mg, about 0.35 mg, or about 0.5 mg once daily. In an embodiment, talazoparib or a pharmaceutically acceptable salt thereof, and preferably a tosylate thereof, is administered at a daily dosage of about 0.25 mg, about 0.35 mg, or about 0.5 mg once daily. In an embodiment, talazoparib or a pharmaceutically acceptable salt thereof and preferably a tosylate thereof, is administered at a daily dosage of about about 0.5 mg, about 0.75 mg or about 1.0 mg once daily. In an embodiment, talazoparib or a pharmaceutically acceptable salt thereof and preferably a tosylate thereof, is administered at a daily dosage of about 0.1 mg once daily. In an embodiment, talazoparib or a pharmaceutically acceptable salt thereof, and preferably a tosylate thereof, is administered at a daily dosage of about 0.25 mg once daily. In an embodiment, talazoparib or a pharmaceutically acceptable salt thereof, and preferably a tosylate thereof, is administered at a daily dosage of about 0.35 mg once daily. In an embodiment, talazoparib or a pharmaceutically acceptable salt thereof, and preferably a tosylate thereof, is administered at a daily dosage of about 0.5 mg once daily. In an embodiment, talazoparib or a pharmaceutically acceptable salt thereof, and preferably a tosylate thereof, is administered at a daily dosage of about 0.75 mg once daily. In an embodiment, talazoparib or a pharmaceutically acceptable salt thereof, and preferably a tosylate thereof, is administered at a daily dosage of about 1.0 mg once daily. Dosage amounts provided herein refer to the dose of the free base form of talazoparib or are calculated as the free base equivalent of an administered talazoparib salt form. For example, a dosage or amount of talazoparib, such as 0.5, 0.75 mg or 1.0 mg refers to the free base equivalent. This dosage regimen may be adjusted to provide the optimal therapeutic response. For example, the dose may be proportionally reduced or increased as indicated by the exigencies of the therapeutic situation.

"Tumor" as it applies to a subject diagnosed with, or suspected of having, a cancer refers to a malignant or potentially malignant neoplasm or tissue mass of any size, and includes primary tumors and secondary neoplasms. A solid tumor is an abnormal growth or mass of tissue that usually does not contain cysts or liquid areas. Examples of solid tumors are sarcomas, carcinomas, and lymphomas. Leukaemia's (cancers of the blood) generally do not form solid tumors (National Cancer Institute, Dictionary of Cancer Terms).

"Tumor burden" also referred to as a "tumor load', refers to the total amount of tumor material distributed throughout the body. Tumor burden refers to the total number of cancer cells or the total size of tumor(s), throughout the body, including lymph nodes and bone marrow. Tumor burden can be determined by a variety of methods known in the art, such as, e.g., using callipers, or while in the body using imaging techniques, e.g., ultrasound, bone scan, computed tomography (CT), or magnetic resonance imaging (MRI) scans.

The term "tumor size" refers to the total size of the tumor which can be measured as the length and width of a tumor. Tumor size may be determined by a variety of methods known in the art, such as, e.g., by measuring the dimensions of tumor(s) upon removal from the subject, e.g., using callipers, or while in the body using imaging techniques, e.g., bone scan, ultrasound, CR or MRI scans.

The methods of the present invention are useful for treating cancer. In some embodiments, the methods provided results in one or more of the following effects: (1) inhibiting cancer cell proliferation; (2) inhibiting cancer cell invasiveness; (3) inducing apoptosis of cancer cells; (4) inhibiting cancer cell metastasis; (5) inhibiting angiogenesis; or (6) overcoming one or more resistance mechanisms relating to a cancer treatment.

In another aspect, this invention relates to a pharmaceutical soft gelatin capsule of the present invention for use in the treatment of cancer in a subject.

In another aspect, this invention relates to a pharmaceutical soft gelatin capsule of the present invention for use as a medicament.

In one embodiment of the invention, the subject is a mammal.

In one embodiment of the invention, the subject is a human.

In some embodiments the methods of the present invention may be useful for the treatment of cancers including but not limited to cancers of the:
circulatory system, for example, heart (sarcoma [angiosarcoma, fibrosarcoma, rhabdomyosarcoma, liposarcoma], myxoma, rhabdomyoma, fibroma, lipoma and teratoma), mediastinum and pleura, and other intrathoracic organs, vascular tumors and tumor-associated vascular tissue,
respiratory tract, for example, nasal cavity and middle ear, accessory sinuses, larynx, trachea, bronchus and lung such as small cell lung cancer (SCLC), non-small cell lung cancer (NSCLC), bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hamartoma, mesothelioma;
gastrointestinal system, for example, esophagus (squamous cell carcinoma, adenocarcinoma, leiomyosarcoma, lymphoma), stomach (carcinoma, lymphoma, leiomyosarcoma), gastric, pancreas (ductal adenocarcinoma, insulinoma, glucagonoma, gastrinoma, carcinoid tumors, vipoma), small bowel (adenocarcinoma, lymphoma, carcinoid tumors, Karposi's sarcoma, leiomyoma, hemangioma, lipoma, neurofibroma, fibroma), large bowel (adenocarcinoma, tubular adenoma, villous adenoma, hamartoma, leiomyoma);
genitourinary tract, for example, kidney (adenocarcinoma, Wilm's tumor [nephroblastoma], lymphoma, leukemia), bladder and/or urethra (squamous cell carcinoma, transitional cell or urothelial carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma);
liver (for example, hepatoma, hepatocellular carcinoma), cholangiocarcinoma, hepatoblastoma, angiosarcoma, hepatocellular adenoma, hemangioma, pancreatic endocrine tumors (such as pheochromocytoma, insulinoma, vasoactive intestinal peptide tumor, islet cell tumor and glucagonoma);
bone, for example, osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors;
nervous system, for example, neoplasms of the central nervous system (CNS), primary CNS lymphoma, skull cancer (osteoma, hemangioma, granuloma, xanthoma, osteitis deformans), meninges (meningioma, meningiosarcoma, gliomatosis), brain cancer (astrocytoma, medulloblastoma, glioma, ependymoma, germinoma [pinealoma], glioblastoma multiform, oligodendroglioma, schwannoma, retinoblastoma, congenital tumors), spinal cord neurofibroma, meningioma, glioma, sarcoma);
reproductive system, for example, gynecological, uterus (endometrial carcinoma), cervix (cervical carcinoma, pre-tumor cervical dysplasia), ovaries (ovarian carcinoma [serous cystadenocarcinoma, mucinous cystadenocarcinoma, unclassified carcinoma], granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), fallopian tubes (carcinoma) and other sites associated with female genital organs; placenta, penis, prostate, testis, and other sites associated with male genital organs;
hematologic system, for example, blood (myeloid leukemia [acute and chronic], acute lymphoblastic leukemia, chronic lymphocytic leukemia, myeloproliferative diseases, multiple myeloma, myelodysplastic syndrome), Hodgkin's disease, non-Hodgkin's lymphoma [malignant lymphoma];
oral cavity, for example, lip, tongue, gum, floor of mouth, palate, and other parts of mouth, parotid gland, and other parts of the salivary glands, tonsil, oropharynx, nasopharynx, pyriform sinus, hypopharynx, and other sites in the lip, oral cavity and pharynx;
skin, for example, malignant melanoma, cutaneous melanoma, basal cell carcinoma, squamous cell carcinoma, Karposi's sarcoma, moles dysplastic nevi, lipoma, angioma, dermatofibroma, and keloids;
adrenal glands: neuroblastoma; and
other tissues including connective and soft tissue, retroperitoneum and peritoneum, eye, intraocular melanoma, and adnexa, breast, head or/and neck, anal region, thyroid, parathyroid, adrenal gland and other endocrine glands and related structures, secondary and unspecified malignant neoplasm of lymph nodes, secondary malignant neoplasm of respiratory and digestive systems and secondary malignant neoplasm of other sites.

In one embodiment, examples of "cancer" when used herein in connection with the present invention include cancer selected from lung cancer (NSCLC and SCLC), breast cancer (including triple negative breast cancer, hormone positive breast cancer, HER2 negative breast cancer, HER2 positive breast cancer and triple positive breast cancer), ovarian cancer, colon cancer, rectal cancer, cancer of the anal region, prostate cancer (including castration-sensitive or hormone sensitive prostate cancer and hormone-refractory prostate cancer, also known as castration-resistant prostate cancer), hepatocellular carcinoma, diffuse large B-cell lymphoma, follicular lymphoma, melanoma and salivary gland tumor or a combination of one or more of the foregoing cancers.

In one embodiment, examples of "cancer" when used herein in connection with the present invention include cancer selected from lung cancer (NSCLC and SCLC), breast cancer (including triple negative breast cancer, hormone positive breast cancer, and HER2 negative breast cancer), ovarian cancer, prostate cancer (including castration-sensitive or hormone sensitive prostate cancer and hormone-refractory prostate cancer, also known as castration-resistant prostate cancer), or a combination of one or more of the foregoing cancers.

In one embodiment, examples of "cancer" when used herein in connection with the present invention include cancer selected from prostate cancer, androgen receptor positive breast cancer, hepatocellular carcinoma, and salivary gland tumor, or a combination of one or more of the foregoing cancers.

In one embodiment, examples of "cancer" when used herein in connection with the present invention include cancer selected from androgen receptor positive breast cancer, hepatocellular carcinoma, and salivary gland tumor, or a combination of one or more of the foregoing cancers.

In one embodiment, examples of "cancer" when used herein in connection with the present invention include cancer selected from triple negative breast cancer, hormone positive breast cancer, HER2 negative breast cancer, triple positive breast cancer, castration-sensitive prostate cancer, castration-resistant prostate cancer, hepatocellular carcinoma, and salivary gland tumor or a combination of one or more of the foregoing cancers.

In one embodiment, examples of "cancer" when used herein in connection with the present invention include cancer selected from triple negative breast cancer, hormone positive breast cancer, and HER2 negative breast cancer, or a combination of one or more of the foregoing cancers.

In one embodiment, examples of "cancer" when used herein in connection with the present invention include cancer selected from castration-sensitive prostate cancer and castration-resistant prostate cancer or a combination of one or more of the foregoing cancers.

In one embodiment of the invention, the cancer is a solid tumor.

In one embodiment of the invention, the cancer is a solid tumor which solid tumor is androgen-dependent.

In one embodiment of the invention, the cancer is a solid tumor which solid tumor expresses androgen receptors.

In one embodiment, the cancer is prostate cancer.

In one embodiment, the cancer is high-risk prostate cancer.

In one embodiment, the cancer is locally advanced prostate cancer.

In one embodiment, the cancer is high-risk locally advanced prostate cancer.

In one embodiment, the cancer is castration-sensitive prostate cancer.

In one embodiment, the cancer is metastatic castration-sensitive prostate cancer.

In one embodiment, the cancer is castration-sensitive prostate cancer or metastatic castration-sensitive prostate cancer with DNA damage repair mutations (DDR mutations). The DDR mutations include ATM, ATR, BRCA1, BRCA2, CHEK2, FANCA, MLH1, MRE11A, NBN, PALB2, and RAD51C.

In one embodiment, the cancer is hormone sensitive prostate cancer, also known as castration sensitive prostate cancer. Hormone sensitive prostate cancer is usually characterized by histologically or cytologically confirmed adenocarcinoma of the prostate which is still responsive to androgen deprivation therapy.

In one embodiment, the cancer is non-metastatic hormone sensitive prostate cancer.

In one embodiment, the cancer is high risk, non-metastatic hormone sensitive prostate cancer.

In one embodiment, the cancer is metastatic hormone sensitive prostate cancer.

In one embodiment, the cancer is castration-resistant prostate cancer, also known as hormone-refractory prostate cancer or androgen-independent prostate cancer. Castration resistant prostate cancer is usually characterized by histologically or cytologically confirmed adenocarcinoma of the prostate which is castration resistant (for example defined as 2 or more consecutive rises of PSA, ≥1 week between each assessment, optionally resulting in 2 or more 50% or greater increases over the nadir, with PSA level ≥2 ng/mL), in a setting of castrate levels of testosterone (for example ≤ 1.7 nmol/L level of testosterone or ≤50 ng/dL level of testosterone), which castrate levels of testosterone are achieved by androgen deprivation therapy and / or post orchiectomy.

In one embodiment, the cancer is non-metastatic castration-resistant prostate cancer.

In one embodiment, the cancer is non-metastatic castration-resistant prostate cancer.

In one embodiment, the cancer is metastatic castration-resistant prostate cancer.

In one embodiment, the cancer is metastatic castration-resistant prostate cancer with DNA repair deficiencies.

In one embodiment, the cancer is breast cancer.

In one embodiment, the cancer is locally advanced or metastatic breast cancer.

In one embodiment, the cancer is triple negative breast cancer.

In one embodiment, the cancer is hormone positive breast cancer, including estrogen positive and / or progesterone positive breast cancer.

In one embodiment, the cancer is HER2 negative breast cancer.

In one embodiment, the cancer is germline BRCA-mutated HER2-negative breast cancer.

In one embodiment, the cancer is HER2 positive breast cancer.

In one embodiment, the cancer is triple positive breast cancer.

In one embodiment, the cancer is ovarian cancer.

In one embodiment, the cancer is small cell lung cancer.

In one embodiment, the cancer is Ewing's sarcoma.

In one embodiment, the cancer is hepatocellular carcinoma.

In one embodiment, the cancer is salivary gland tumor.

In one embodiment, the cancer is locally advanced.

In one embodiment, the cancer is non-metastatic.

In one embodiment, the cancer is metastatic.

In one embodiment, the cancer is refractory.

In one embodiment, the cancer is relapsed.

In one embodiment, the cancer is intolerable of standard treatment.

In one embodiment, the cancer has a CDK12 mutation.

In one embodiment of the present invention, the method is administered to a subject diagnosed with cancer, which cancer has developed resistance to treatment.

In a further aspect, the methods of the present invention may additionally comprise administering further anti-cancer agents, such as anti-tumor agents, anti-angiogenesis agents, signal transduction inhibitors and antiproliferative agents, which amounts are together effective in treating said cancer. In some such embodiments, the anti-tumor agent is selected from the group consisting of mitotic inhibitors, alkylating agents, anti-metabolites, intercalating antibiotics, growth factor inhibitors, radiation, cell cycle inhibitors, enzymes, topoisomerase inhibitors, biological response modifiers, antibodies, cytotoxics, anti-hormones, androgen deprivation therapy and antiandrogens. In an embodiment of the present invention, the further anti-cancer agent is an anti-androgen. In an embodiment of the present invention, the anti-androgen is enzalutamide or apalutamide.

### Examples

### Example 1: Preparation of Talazoparib 0.1 mq, 0.25 mq, 0.35 mq, 0.5 mq, 0.75 mg and 1 mg Soft Gelatin Capsules

The composition of the pharmaceutical soft gelatin capsules as 0.1 mg, 0.25 mg, 0.35 mg, 0.5 mg, 0.75 mg and 1 mg dosage forms of talazoparib, including the compositions of the fill and the shell are set forth below in Tables 1-6, respectively.

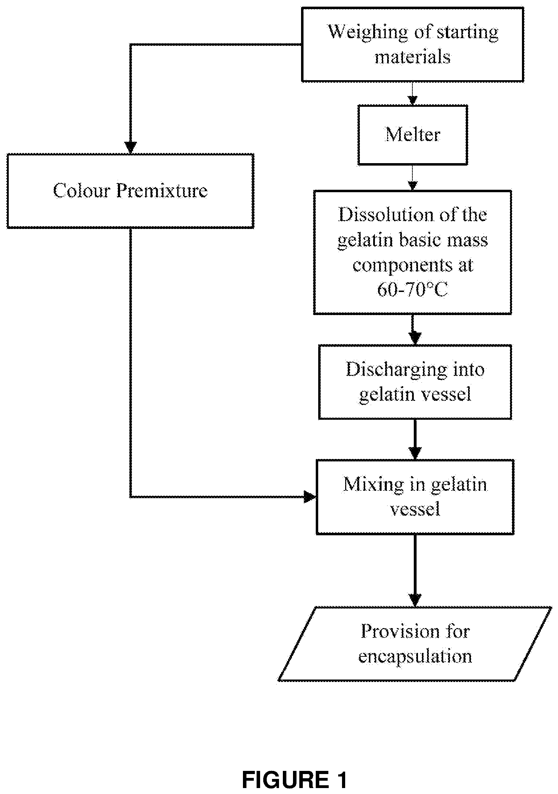

The flow diagram of the manufacturing process for the gelatin mass of pharmaceutical soft gelatin capsules as 0.1 mg, 0.25 mg, 0.35 mg, 0.5 mg, 0.75 mg and 1 mg dosage forms of talazoparib is provided in FIGURE 1. As shown in FIGURE 1, the components of the gelatin mass were mixed and dissolved at 60-70°C. The desired colors were added and the gelatin mass was provided for encapsulation, as described below.

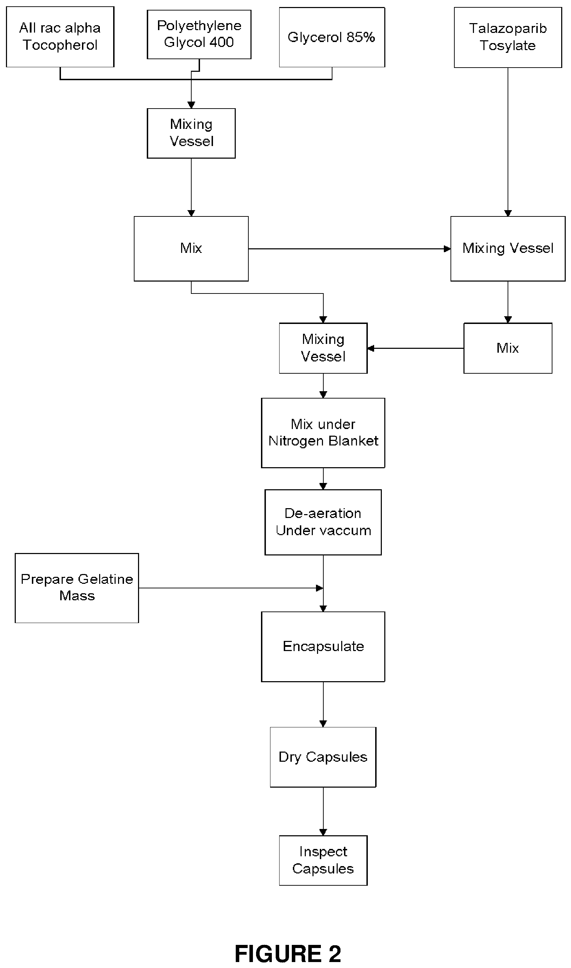

The flow diagram of the manufacturing process for the fill solution and encapsulation of pharmaceutical soft gelatin capsules as 0.1 mg, 0.25 mg, 0.35 mg, 0.5 mg, 0.75 mg and 1 mg dosage forms of talazoparib is provided in FIGURE 2 and set forth in the numbered steps below. The item numbers correspond to the components in Tables 1-6 below.
1. A suitable mixing vessel was charged with polyethylene glycol 400 (item 2), glycerol 85% (item 3) and all-rac-alpha-tocopherol (item 4) in a suitable mixing vessel and mixed.
2. Talazoparib tosylate (item 1) was mixed with a portion of the mixture from step 1.
3. Half of the mixture from step 1 and the mixture from step 2 were transferred into a suitable mixing unit. The mixing vessel from step 2 was rinsed three times with the mixture from step 1 to ensure that all the talazoparib tosylate (item 1) was transferred into the mixing unit. The remaining mixture from step 1 was added to the suitable mixing unit.
4. The contents from step 3 were mixed.
5. The solution from step 4 was de-aerated at permanent vacuum.
6. The solution from step 5 was discharged from the mixing tank directly to the fill vessel of the encapsulation machine.
7. The gelatin mass, prepared as described above, was utilized to encapsulate the capsules.
8. The resulting capsules were dried in tunnels on trays.
9. After drying, the capsules were visually inspected.

**Table 1 (composition with acid bone gelatin is according to the invention)**

| **Soft Gel Capsule Composition - 0.1 mg Talazoparib Capsules** | | | |
|---|---|---|---|
| | **Component** | **Quantity/Unit: (mg/capsule)** | **Percent by Weight** |
| | **Capsule Fill** | | |
| 1 | Talazoparib Tosylate | 0.145 ^{a} | 0.145 |
| 2 | Polyethylene Glycol 400 | 95.555 | 95.555 |
| 3 | Glycerol 85% | 4.000 | 4.00 |
| 4 | All-rac-alpha-Tocopherol | 0.300 | 0.30 |
| | **Total for Capsule Fill** | 100.000 | 100 |
| | | | |

| | **Gelatin Mass** | | |
|---|---|---|---|
| 5 | Gelatin (acid hide gelatin 195 Bloom or acid bone gelatin 195 Bloom) | 46.270 | 60.73 |
| 6 | Glycerol 85% | 11.790 | 15.47 |
| 7 | Dry Substance of Sorbitol Liquid, partially dehydrated | 17.57 | 23.06 |
| 8 | Dye(s) | 0.560 | 0.74 |
| | | | |
| | **Total for Gelatin Mass** | 76.190 ^{c} | 100 |
| 9 | Purified Water ^{b} | 34.800 - 38.800 | q.s. ^{d} |

| **Footnotes:** | | | |
|---|---|---|---|
| ^{a} Equivalent to 0.1 mg of talazoparib free base per capsule based on an assay value of 100% for talazoparib tosylate | | | |
| ^{b} Total water for dissolution of gelatin and for color mix | | | |
| ^{c} Total calculated as the dried capsule shell weight based on a ribbon thickness of 30 1/1000 inch | | | |
| ^{d} Water in sufficient quantity to dissolve the gelatin mass | | | |

**Table 2 (composition with acid bone gelatin is according to the invention)**

| **Soft Gel Capsule Composition - 0.25 mg Talazoparib Capsules** | | | |
|---|---|---|---|
| | **Component** | **Quantity/Unit: (mg/capsule)** | **Percent by Weight** |
| | **Capsule Fill** | | |
| 1 | Talazoparib Tosylate | 0.363 ^{a} | 0.29 |
| 2 | Polyethylene Glycol 400 | 119.262 | 95.41 |
| 3 | Glycerol 85% | 5.000 | 4.00 |
| 4 | All-rac-alpha-Tocopherol | 0.375 | 0.30 |
| | **Total for Capsule Fill** | 125.000 | 100 |
| | | | |

| | **Gelatin Mass** | | |
|---|---|---|---|
| 5 | Gelatin (acid hide gelatin 195 Bloom or acid bone gelatin 195 Bloom) | 61.097 | 59.77 |
| 6 | Glycerol 85% | 16.610 | 16.25 |
| 7 | Dry Substance of Sorbitol Liquid, partially dehydrated | 23.190 | 22.68 |
| 8 | Dye(s) | 1.330 | 1.30 |
| | **Total for Gelatin Mass** | 102.227 ^{c} | 100 |
| 9 | Purified Water ^{b} | 45.700 - 52.700 | q.s. ^{d} |

| **Footnotes:** | | | |
|---|---|---|---|
| ^{a} Equivalent to 0.25 mg of talazoparib free base per capsule based on an assay value of 100% for talazoparib tosylate | | | |
| ^{b} Total water for dissolution of gelatin and for color mix | | | |
| ^{c} Total calculated as the dried capsule shell weight based on a ribbon thickness of 30 1/1000 inch | | | |
| ^{d} Water in sufficient quantity to dissolve the gelatin mass | | | |

**Table 3 (composition with acid bone gelatin is according to the invention)**

| **Soft Gel Capsule Composition - 0.35 mg Talazoparib Capsules** | | | |
|---|---|---|---|
| | **Component** | **Quantity/Unit: (mg/capsule)** | **Percent by Weight** |
| | **Capsule Fill** | | |
| 1 | Talazoparib Tosylate | 0.509 a | 0.29 |
| 2 | Polyethylene Glycol 400 | 166.966 | 95.41 |
| 3 | Glycerol 85% | 7.000 | 4.00 |
| 4 | All-rac-alpha-Tocopherol | 0.525 | 0.30 |
| | **Total for Capsule Fill** | 175.000 | 100 |
| | | | |

| | **Gelatin Mass** | | |
|---|---|---|---|
| 5 | Gelatin (acid hide gelatin 195 Bloom or acid bone gelatin 195 Bloom) | 78.130 | 60.44 |
| 6 | Glycerol 85% | 20.250 | 15.67 |
| 7 | Dry Substance of Sorbitol Liquid, partially dehydrated | 29.66 | 22.94 |
| 8 | Dye(s) | 1.227 | 0.95 |
| | **Total for Gelatin Mass** | 129.267 ^{c} | 100 |
| 9 | Purified Water^{b} | 58.600 - 65.800 | q.s. ^{d} |

| **Footnotes:** | | | |
|---|---|---|---|
| ^{a} Equivalent to 0.35 mg of talazoparib free base per capsule based on an assay value of 100% for talazoparib tosylate | | | |
| ^{b} Total water for dissolution of gelatin and for color mix | | | |
| ^{c} Total calculated as the dried capsule shell weight based on a ribbon thickness of 30 1/1000 inch | | | |
| ^{d} Water in sufficient quantity to dissolve the gelatin mass | | | |

**Table 4 (composition with acid bone gelatin is according to the invention)**

| **Soft Gel Capsule Composition - 0.5 mg Talazoparib Capsules** | | | |
|---|---|---|---|
| | **Component** | **Quantity/Unit: (mg/capsule)** | **Percent by Weight** |
| | **Capsule Fill** | | |
| 1 | Talazoparib Tosylate | 0.727 a | 0.29 |
| 2 | Polyethylene Glycol 400 | 238.523 | 95.41 |
| 3 | Glycerol 85% | 10.000 | 4.00 |
| 4 | All-rac-alpha-Tocopherol | 0.750 | .30 |
| | **Total for Capsule Fill** | 250.000 | 100 |
| | | | |

| | **Gelatin Mass** | | |
|---|---|---|---|
| 5 | Gelatin (acid hide gelatin 195 Bloom or acid bone gelatin 195 Bloom) | 104.930 | 60.87 |
| 6 | Glycerol 85% | 26.640 | 15.45 |
| 7 | Dry Substance of Sorbitol Liquid, partially dehydrated | 39.830 | 23.10 |
| 8 | Dye(s) | 0.994 | 0.58 |
| | **Total for Gelatin Mass** | 172.394 ^{c} | 100 |
| 9 | Purified Water ^{b} | 78.700 - 90.000 | q.s. ^{d} |

| **Footnotes:** | | | |
|---|---|---|---|
| ^{a} Equivalent to 0.5 mg of talazoparib free base per capsule based on an assay value of 100% for talazoparib tosylate | | | |
| ^{b} Total water for dissolution of gelatin and for color mix | | | |
| ^{c} Total calculated as the dried capsule shell weight based on a ribbon thickness of 32 1/1000 inch | | | |
| ^{d} Water in sufficient quantity to dissolve the gelatin mass | | | |

**Table 5 (composition with acid bone gelatin is according to the invention)**

| **Soft Gel Capsule Composition - 0.75 mg Talazoparib Capsules** | | | |
|---|---|---|---|
| | **Component** | **Quantity/Unit: (mg/capsule)** | **Percent by Weight** |
| | **Capsule Fill** | | |
| 1 | Talazoparib Tosylate | 1.091 ^{a} | 0.2909 |
| 2 | Polyethylene Glycol 400 | 357.784 | 95.4091 |
| 3 | Glycerol 85% | 15.000 | 4.00 |
| 4 | All-rac-alpha-Tocopherol | 1.125 | 0.30 |
| | **Total for Capsule Fill** | 375.000 | 100 |
| | | | |

| | **Gelatin Mass** | | |
|---|---|---|---|
| 5 | Gelatin (acid hide gelatin 195 Bloom or acid bone gelatin 195 Bloom) | 141.260 | 60.73 |
| 6 | Glycerol 85% | 36.000 | 15.48 |
| 7 | Dry Substance of Sorbitol Liquid, partially dehydrated | 53.620 | 23.05 |
| 8 | Dyes | 1.710 | 0.74 |
| | **Total for Gelatin Mass** | 232.590 ^{c} | 100 |
| 9 | Purified Water ^{b} | 106.200 - 121.600 | q.s ^{d} |

| **Footnotes:** | | | |
|---|---|---|---|
| ^{a} Equivalent to 0.75 mg of talazoparib free base per capsule based on an assay value of 100% for talazoparib tosylate | | | |
| ^{b}Total water for dissolution of gelatin and for colour mix | | | |
| ^{c} Total calculated as the dried capsule shell weight based on a ribbon thickness of 32 1/1000 inch | | | |
| ^{d} Water in sufficient quantity to dissolve the gelatin mass | | | |

**Table 6 (composition with acid bone gelatin is according to the invention)**

| **Soft Gel Capsule Composition - 1 mg Talazoparib Capsules** | | | |
|---|---|---|---|
| | **Component** | **Quantity/Unit: (mg/capsule)** | **Percent by Weight** |
| | **Capsule Fill** | | |
| 1 | Talazoparib Tosylate | 1.453 ^{a} | 0.29 |
| 2 | Polyethylene Glycol 400 | 477.047 | 95.41 |
| 3 | Glycerol 85% | 20.000 | 4.00 |
| 4 | All-rac-alpha-Tocopherol | 1.500 | 0.30 |
| | **Total for Capsule Fill** | 500.000 | 100 |
| | | | |

| | **Gelatin Mass** | | |
|---|---|---|---|
| 5 | Gelatin (acid hide gelatin 195 Bloom or acid bone gelatin 195 Bloom) | 177.790 | 60.73 |
| 6 | Glycerol 85% | 45.310 | 15.48 |
| 7 | Dry Substance of Sorbitol Liquid, partially dehydrated | 67.490 | 23.05 |
| 8 | Dye(s) | 2.160 | 0.74 |
| | **Total for Gelatin Mass** | 292.750 ^{c} | 100 |
| 9 | Purified Water ^{b} | 142.1 - 160.8 | q.s. ^{d} |

| **Footnotes:** | | | |
|---|---|---|---|
| ^{a} Equivalent to 1 mg of talazoparib free base per capsule based on an assay value of 100% for talazoparib tosylate | | | |
| ^{b} Total water for dissolution of gelatin and for color mix | | | |
| ^{c} Total calculated as the dried capsule shell weight based on a ribbon thickness of 32 1/1000 inch | | | |
| ^{d} Water in sufficient quantity to dissolve the gelatin mass | | | |

One of ordinary skill in pharmaceutical manufacturing would understand that the values provided in Tables 1-6 are theoretical formulations for a single unit (the QQ formula (Qua Que)). When a pharmaceutical product is scaled up to multiple units, the quantity for each component is multiplied by the number of units required. Due to differences in balance accuracy and tolerances, in calculating back from the weights used to manufacture a batch, there is a potential for slight differences in the QQ values. Additionally, during manufacturing, certain limits are allowed on the accuracy of filling the capsules and the weight of the capsule shell, however, as a percentage of the weight measured, these values will be the same as the QQ formula. This is recognized within the industry and with the regulators.

### Example 2: Acid Bone Gelatin Demonstrated Improved Chemical Stability Compared to Acid Hide Gelatin in Talazoparib Soft Gelatin Capsules

The impact of the type of gelatin, acid hide versus acid bone, utilized in the preparation of talazoparib soft gelatin capsules was evaluated to determine the effect on chemical stability of the capsule formulations. The manufacturer, Gelita, was the supplier of the acid hide and acid bone gelatin. Representative batches of talazoparib soft gelatin capsules, as shown in Table 7, using both gelatin types were manufactured and assessed for chemical stability (0.1 mg capsule formulation and 1 mg capsule formulation as 30 count in 60 cc high density polyethylene (HDPE) bottles).

**Table 7 (composition with acid bone gelatin is according to the invention)**

| **Strength** | **Batch No.** | **Gelatin Source** | **Gelatin Type** | **Gelatin Lot No.** |
|---|---|---|---|---|
| 0.1 mg | 1 | Acid Hide | A | A |
| | 2 | Acid Hide | A | B |
| | 3 | Acid Bone | A | C |
| | 8 | Acid Bone | A | G |
| 1 mg | 4 | Acid Hide | A | A |
| | 5 | Acid Hide | A | D |
| | 6 | Acid Bone | A | E |
| | 7 | Acid Bone | A | C |
| | 9 | Acid Bone | A | F |

The representative batches were prepared using the general procedure of Example 1 and the general compositions of Tables 1 and 6 with the following substantive modifications. Batch No. 1 was prepared using 0.5% tocopherol and no glycerol 85% in the capsule fill; and no glycerol 85% in gelatin mass. Batch No. 4 was prepared using 0.5% tocopherol and no glycerol 85% in the capsule fill. Batches No. 2, 3, 5, 6, 7, 8 and 9 were prepared using 0.3% tocopherol and 4% glycerol 85% in the liquid fill; and 11% glycerol 85% in gelatin mass.

Capsules were set on stability under accelerated conditions, 40°C/75% relative humidity ("RH") and long-term conditions 30°C/75% RH. The degradation product, the cis-isomer of talazoparib ("cis talazoparib"), is the shelf life limiting degradation product and is shown below.

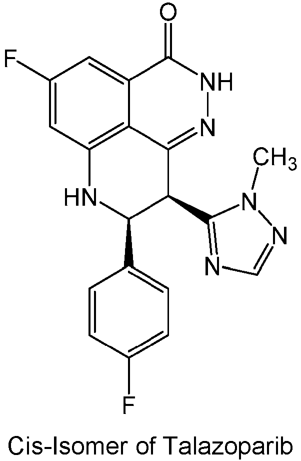

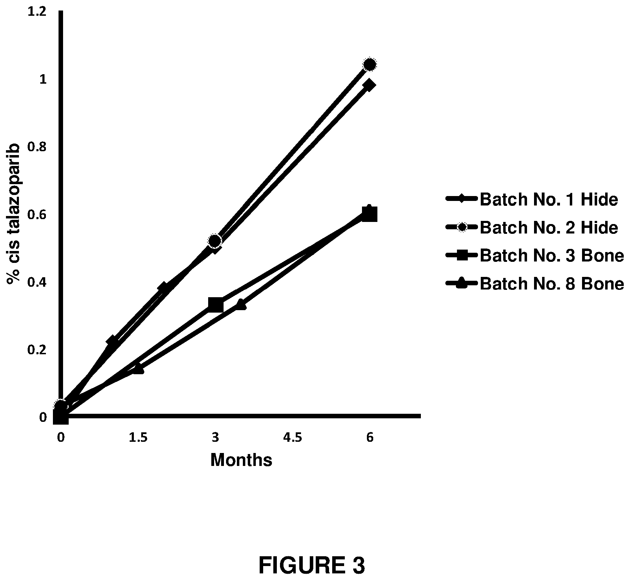

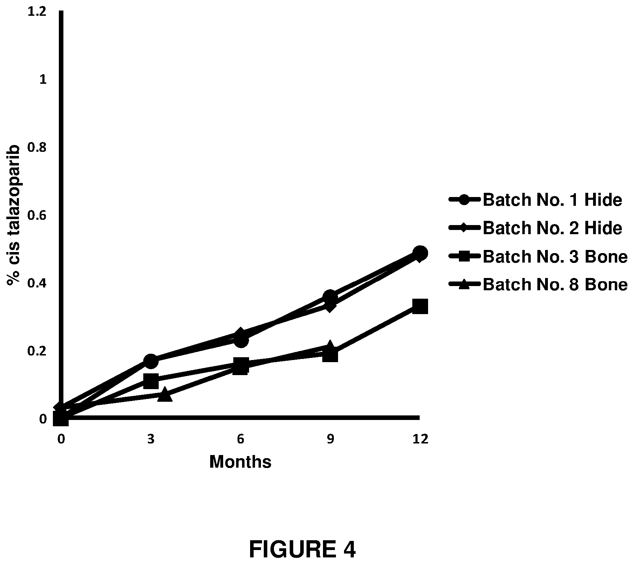

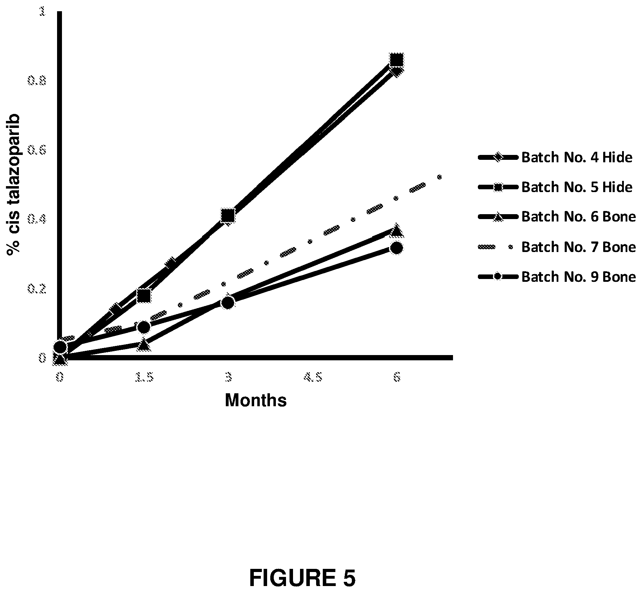

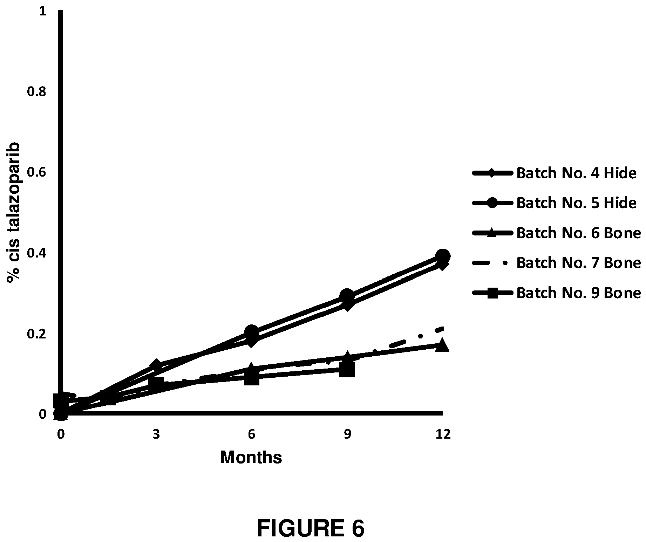

The chemical stability of the capsule formulations stored at 40°C/75% RH and 30°C/75% RH are provided in FIGURE 3 to FIGURE 6 and Tables 8 and 9. FIGURES 3 and 4 demonstrate a lower degradation rate for two batches of 0.1 mg formulation manufactured from different lots of acid bone compared to two batches manufactured with different lots of acid hide gelatin. Similarly, FIGURES 5 and 6 show that 1 mg batches manufactured with different lots of acid bone gelatin had a lower rate of formation of cis talazoparib compared to batches manufactured with different lots of acid hide gelatin. The raw data for FIGURE 3 to FIGURE 6 are shown in Tables 8 and 9 below.

**Table 8: Chemical Stability at 30°C/75% RH of Representatives Batches of Talazoparib Soft Gelatin Capsules**

| | | | **Cis Talazoparib (%)** | | | | | |
|---|---|---|---|---|---|---|---|---|
| **Strength** | **Batch No.** | **Gelatin Source** | **Initial** | **1.5 Months** | **3 Months** | **6 Months** | **9 Months** | **12 Months** |
| 0.1 mg | 1 | Acid Hide | 0 | NT | 0.17 | 0.23 | 0.36 | 0.49 |
| | 2 | Acid Hide | 0.03 | NT | 0.17 | 0.25 | 0.33 | 0.48 |
| | 3 | Acid Bone | 0 | NT | 0.11 | 0.16 | 0.19 | 0.33 |
| | 8 | Acid Bone | 0.03 | NT | 0.07 | 0.15 | 0.21 | NT |
| 1 mg | 4 | Acid Hide | 0 | NT | 0.12 | 0.18 | 0.27 | 0.37 |
| | 5 | Acid Hide | 0 | NT | NT | 0.20 | 0.29 | 0.39 |
| | 6 | Acid Bone | 0 | NT | NT | 0.11 | 0.14 | 0.17 |
| | 7 | Acid Bone | 0.05 | 0.03 | 0.07 | 0.12 | 0.13 | 0.21 |
| | 9 | Acid Bone | 0.03 | 0.04 | 0.07 | 0.06 | 0.11 | NT |

| | | | | | | | | |
|---|---|---|---|---|---|---|---|---|
| NT: Not Tested | | | | | | | | |

**Table 9: Chemical stability at 40°C/75% RH of Representatives Batches of Talazoparib Soft Gelatin Capsules**

| | | | **Cis Talazoparib (%)** | | | | |
|---|---|---|---|---|---|---|---|
| **Strength** | **Batch No.** | **Gelatin Source** | **Initial** | **1 Month** | **2 Months** | **3 Months** | **6 Months** |
| 0.1 mg | 1 | Acid Hide | 0 | 0.22 | 0.38 | 0.50 | 0.98 |
| | 2 | Acid Hide | 0.03 | NT | NT | 0.52 | 1.04 |
| | 3 | Acid Bone | 0 | NT | NT | 0.33 | 0.6 |
| | 8 | Acid Bone | 0.03 | 0.14* | NT | 0.33 | 0.61 |
| 1 mg | 4 | Acid Hide | 0 | 0.14 | 0.27 | 0.40 | 0.83 |
| | 5 | Acid Hide | 0 | 0.18* | NT | 0.41 | 0.86 |
| | 6 | Acid Bone | 0 | 0.04* | NT | 0.17 | 0.37 |
| | 7 | Acid Bone | 0.05 | 0.10* | NT | 0.22 | 0.54 |
| | 9 | Acid Bone | 0.03 | 0.09* | NT | 0.16 | 0.32 |

| | | | | | | | |
|---|---|---|---|---|---|---|---|
| NT: Not Tested *: tested at 1.5 months | | | | | | | |

As per the ICH Harmonised Tripartite Guideline "Impurities in New Drug Products" Q3B (R2) dated 2 June 2006, an end of shelf life threshold of 1.0 % for cis talazoparib has been qualified.

A significantly slower rate of talazoparib degradation was observed in soft gelatin capsules prepared with acid bone gelatin versus acid hide gelatin when stored under accelerated (40 °C/75% relative humidity) and long term (30 °C/75% relative humidity) conditions. This finding was consistent across different lots of acid bone and acid hide gelatin.

Further, this finding was consistent across formulations having different levels of drug loading or strengths, e.g., 0.1 mg fill formulation has lower drug loading compared to the 1 mg fill formulation). The ratio of the components in the gelatin mass were comparable across all strengths (e.g., 0.1 mg, 0.25 mg, 0.35 mg, 0.5 mg, 0.75 mg and 1 mg dosage forms of talazoparib). There were some slight differences due to the amount of dye used in each formulation; however, the overall gelatin mass was considered equivalent. The capsule fill was the same for all strengths, with the exception that the amount of talazoparib tosylate varied for the 0.1 mg compared with the 0.25 mg, 0.35 mg, 0.5 mg, 0.75 mg and 1 mg dosage forms.

### Example 3: Acid Bone Gelatin Demonstrated Improved Chemical Stability Compared to Acid Hide Gelatin or Limed Bone Gelatin in Talazoparib Soft Gelatin Capsules

The impact of the type of gelatin, specifically, acid hide, acid bone and limed bone, from different suppliers was evaluated to determine the effect on chemical stability of the talazoparib soft gelatin capsules. Representative batches of talazoparib soft gelatin capsules, as shown in the first four columns of Table 10, using different gelatin types were manufactured and assessed for chemical stability (1 mg capsule formulation as 30 count in 60 cc HDPE bottles with heat induction seal).

**Table 10: Representative Batches of 1 mg Talazoparib Soft Gelatin Capsules (composition with acid bone gelatin is according to the invention)**

| **Batch No.** | **Gelatin Source** | **Gelatin Type** | **Gelatin Manufacturer** |
|---|---|---|---|
| 1 | Limed Bone | B | Gelita |
| 2 | Limed Bone | B | Gelita |
| 3 | Limed Bone | B | Rousselot |
| 4 | Limed Bone | B | Rousselot |
| 5 | Acid Bone | A | PB Leiner UK |
| 6 | Acid Bone | A | PB Leiner UK |
| 7 | Acid Hide | A | PB Leiner UK |
| 8 | Acid Bone | A | PB Leiner UK |
| 9 | Acid Bone | A | PB Leiner UK |
| 10 | Acid Bone | A | Rousselot |

The representative batches were prepared using the general procedure of Example 1 and the general compositions of Tables 1 and 6. Batch Nos. 1 and 2 were prepared using 150 Limed Bone SRM free gelatin from Gelita. Batch Nos. 3 and 4 were prepared using 150 Limed Bone gelatin from Rousselot. Batch Nos. 5 and 6 were prepared using 195 Acid Bone SRM free gelatin from PB Leiner UK. Batch No. 7 was prepared using 195 Acid Hide gelatin from PB Leiner UK. Batch Nos. 8 and 9 were prepared using 195 Acid Bone Vertebrae free gelatin from PB Leiner UK. Batch No. 10 was prepared using 195 Acid Bone Vertebrae free from Rousselot. The only differences between the shell mass used for the different batches was the gelatin source used. All the remaining components (glycerol ,sorbitol and pigments) remained the same at the same ratios for the dosage strength evaluated.

The overall study design (storage conditions and time points) is based on ICH Harmonised Tripartite Guideline "Stability Testing Of New Drug Substances And Products" Q1A (R2), dated 6 February 2003, criteria for long-term and accelerated conditions for a twelve-month period. Capsules were set on stability under long-term conditions, 30°C/75% RH, and accelerated conditions, 40°C/75% RH. The degradation product, cis talazoparib, is the shelf life limiting degradation product. The initial stability of the capsule formulations and the capsule formulations stored at 40°C/75% RH and 30°C/75% RH at 6 month and 9 month timepoints are provided in Tables 11 and 12, respectively.

**Table 11: Chemical Stability at 30°C/75% RH of Representative Batches of 1 mg Talazoparib Soft Gelatin Capsules**

| **Batch No.** | **Gelatin Source / Type / Manufacturer** | **Cis Talazoparib (%)** | | |
|---|---|---|---|---|
| | | **Initial** | **6 Month** | **9 Month** |
| 1 | Limed Bone / B / Gelita | ND | 0.18 | 0.27 |
| 2 | Limed Bone / B / Gelita | ND | 0.15 | 0.23 |
| 3 | Limed Bone / B / Rousselot | ND | 0.19 | 0.27 |
| 4 | Limed Bone / B / Rousselot | ND | 0.19 | 0.29 |
| 5 | Acid Bone / A / PB Leiner UK | ND | NMT 0.1 | 0.12 |
| 6 | Acid Bone / A / PB Leiner UK | ND | 0.11 | 0.16 |
| 7 | Acid Hide / A / PB Leiner UK | NMT 0.1 | 0.24 | |
| 8 | Acid Bone / A / PB Leiner UK | NMT 0.1 | 0.09 | |
| 9 | Acid Bone / A / PB Leiner UK | NMT 0.1 | 0.10 | |
| 10 | Acid Bone / A / Rousselot | NMT 0.1 | 0.09 | |

| | | | | |
|---|---|---|---|---|
| ND: Not detected NMT: Not more than | | | | |

**Table 12: Chemical Stability at 40°C/75% RH of Representative Batches of 1 mg Talazoparib Soft Gelatin Capsules**

| **Batch No.** | **Gelatin Source / Process / Manufacturer** | **Cis Talazoparib (%)** | | | |
|---|---|---|---|---|---|
| | | **Initial** | **6 Week** | **3 Month** | **6 Month** |
| 1 | Limed Bone / B / Gelita | ND | 0.19 | 0.40 | 0.88 |
| 2 | Limed Bone / B / Gelita | ND | 0.17 | 0.35 | 0.75 |
| 3 | Limed Bone / B / Rousselot | ND | 0.19 | 0.40 | 0.86 |
| 4 | Limed Bone / B / Rousselot | ND | 0.20 | 0.42 | 0.91 |
| 5 | Acid Bone / A / PB Leiner UK | ND | NMT 0.1 | 0.16 | 0.30 |
| 6 | Acid Bone / A / PB Leiner UK | ND | 0.13 | 0.22 | 0.45 |
| 7 | Acid Hide / A / PB Leiner UK | NMT 0.1 | 0.24 | 0.51 | 1.0 |
| 8 | Acid Bone / A / PB Leiner UK | NMT 0.1 | NMT 0.1 | 0.16 | 0.30 |
| 9 | Acid one / A / PB Leiner UK | NMT 0.1 | NMT 0.1 | 0.18 | 0.35 |
| 10 | Acid Bone / A / Rousselot | NMT 0.1 | NMT 0.1 | 0.15 | 0.29 |

| | | | | | |
|---|---|---|---|---|---|
| ND: Not detected NMT: Not more than | | | | | |

The chemical stability results reported in Tables 11 and 12 demonstrate lower degradation for all batches manufactured of acid bone gelatin irrelevant of supplier.

### Example 4: A Phase 1 Bioequivalence Study Between the Current Commercial Formulation and the Soft Gelatin Capsule Formulation of Talazoparib, and Food Effect Study for the Talazoparib Soft Gelatin Capsule Formulation in Patients with Advanced Solid Tumors

A Phase 1, open label, 2-sequence, crossover study will be conducted to establish the bioequivalence ("BE") of the current commercial formulation of talazoparib capsules to the talazoparib liquid-filled soft gelatin capsule formulation after multiple dosing under fasting conditions in patients with advanced solid tumors, solid tumors, ovarian cancer, breast cancer, prostate cancer, NSCLC, pancreatic cancer and colorectal cancer. In addition, the effect of food on the pharmacokinetics ("PK") of the talazoparib soft gel capsule formulation will be evaluated in fixed sequence after the 2 BE assessment periods.

### Study Design:

Patients will be randomly assigned to 1 of 2 sequences to receive Treatment A, B and C in different order as shown below. The first 2 periods will be for BE assessment, with the first period being 28 days and the following periods being 21 days. Period 3 will be a 21 day period to evaluate the food effect on the PK of the proposed talazoparib soft gelatin capsule formulation that will be included in the fixed sequence after the 2 BE assessment periods (for patients who can tolerate one high-fat/high-calorie meal). Patients must have received 21 consecutive days of continuous 1 mg once daily drug administration to be considered as completers of a treatment period, before moving on to the next scheduled treatment. The study design is shown in Table 13 below.

**Table 13**

| Arms | Assigned Interventions |
|---|---|
| Experimental: Sequence 1 | Drug: Treatment A |
| Patients receive Treatment B for 28 days, followed by Treatment A for 21 days, followed by Treatment C for 21 days. | Current commercial talazoparib formulation 1 mg once daily given under fasting condition |
| | Drug: Treatment B |
| | talazoparib soft gel capsule formulation 1 mg once daily under fasting condition |
| | Drug: Treatment C talazoparib soft gel capsule formulation 1 mg once daily under fed condition |
| Experimental: Sequence 2 Patients receive Treatment A for 28 days, followed by Treatment B for 21 days, followed by Treatment C for 21 days. | Drug: Treatment A |
| | Current commercial talazoparib formulation 1 mg once daily given under fasting condition |
| | Drug: Treatment B |
| | talazoparib soft gel capsule formulation 1 mg once daily under fasting condition |
| | Drug: Treatment C |
| | talazoparib soft gel capsule formulation 1 mg once daily under fed condition |

### Primary Outcome Measures:

- AUC₂₄ of all talazoparib treatment [ Time Frame: On the last day of each treatment period ]
   Area under the plasma concentration-time curve from time 0 to 24 hours
- Cₘₐₓ of all talazoparib treatment [ Time Frame: On the last day of each treatment period ]
   Maximum plasma concentration

### Secondary Outcome Measures:

- Tₘₐₓ of all talazoparib treatment [ Time Frame: on the last day of treatment period ] Time for Cmax
- C_{trough} of all talazoparib treatment [ Time Frame: on the last day of treatment period ] Predose plasma drug concentration
- CL/F of all talazoparib treatment [ Time Frame: on the last day of treatment period ] Apparent clearance after oral dose
- AUCₗₐₛₜ of all talazoparib treatment [ Time Frame: on the last day of treatment period ]
   Area under the plasma concentration-time profile from time zero to the time of the last quantifiable concentration (Clast)
- Safety and tolerability of the proposed talazoparib soft gel capsule formulation [ Time Frame: Approximately 4 years ]
   Incidence of Aes characterized by type, severity (graded by NCI Common Terminology Criteria for Adverse Events ("CTCAE") version 5.0), timing, seriousness and relationship to study treatment

### Criteria

### Inclusion Criteria:

1. Histological diagnosis of recurrent, locally advanced or metastatic solid tumor that is not amenable for treatment with curative intent.
   - Solid tumors with known or likely pathogenic germline or somatic tumor gene defect (eg, one or more BRCA1 or BRCA2 gene defect except for ovarian cancer) that would benefit from PARPi therapy per current approvals for the tumor indication or supported by strong scientific evidence.
   - Received at least 1 prior SOC regimen, if it exists, as appropriate for the respective tumor type unless deemed unsuitable or declined these therapies; ovarian cancer patients must have at least 1 prior cytotoxic chemotherapy regimen, including at least 1 line of platinum-based therapy. Patients (except for those with ovarian cancer) must not have had disease progression within 6 months of initiation of platinum containing regimen.
2. Eastern Cooperative Oncology Group ("ECOG") performance score of 0-1.
3. Adequate organ function:
   - Absolute neutrophil count ("ANC") ≥1500 cells/mm3
   - Platelets ≥100,000 cells/mm3
   - Hemoglobin ≥10.0 g/dL
   - Creatine clearance ("CLCR") ≥60 mL/min and no documented CLCR <60 mL/min and no change in CLCR >25% in the past 4 weeks
   - Aspartate aminotransferase ("AST") and alanine aminotransferase ("ALT") ≤2.5 × upper limit of normal ("ULN"); if liver function abnormalities are due to hepatic metastasis, then AST and ALT ≤5 × ULN;
   - Total bilirubin ≤1.5 × ULN (≤3 × ULN for Gilbert's syndrome);

### Exclusion Criteria:

1. For ovarian patients: Non-epithelial tumors or ovarian tumors with low malignant potential (ie, borderline tumors) or mucinous tumors.
2. Toxicities from previous anti-cancer therapies must be resolved to NCI CTCAE <Grade 2, except for alopecia, sensory neuropathies ≤Grade 2, or other Grade ≤2 Aes not constituting a safety risk, based on investigator's judgment, are acceptable.
3. Diagnosed with myelodyplastic syndrome ("MDS") or acute myeloid leukemia ("AML").
4. Active infection requiring systemic therapy within 2 weeks of enrollment.
5. Any condition in which active bleeding or pathological conditions may carry a high risk of bleeding (eg, known bleeding disorder, coagulopathy or tumor involvement with major vessels).
6. Known or suspected brain metastasis or active leptomeningeal disease undergoing or requiring treatment. Asymptomatic brain metastases currently not undergoing treatment are allowed.
7. Known history of testing positive for HIV, AIDS, positive hepatitis B virus ("HBV") surface antigen, positive hepatitis virus ("HCV") RNA, or positive COVID-19 viral test. Asymptomatic patients with no active infection detected but positive antibody tests, indicating past infection, are allowed.
8. Current or anticipated use of P-gp inhibitors, BCRP inhibitors, and P-gp inducers within 2 weeks or 5 half-lives prior to randomization (whichever is longer).

### Statistical Methods

To assess BE, natural log transformed AUC₂₄ and Cₘₐₓ after multiple dosing on the last day of treatment period 1 and 2 will be analyzed using a mixed-effect model with sequence, period, and treatment as fixed effects and participant within sequence as a random effect. Estimates of the adjusted mean differences (Test-Reference) and corresponding 90% confidence intervals will be obtained from the model. The adjusted mean differences and 90% confidence intervals for the differences will be exponentiated to provide estimates of the ratio of adjusted geometric means (Test/Reference) and 90% confidence intervals for the ratios. Treatment A (commercial formulation given under fasting condition) will be the Reference treatment while Treatment B (the proposed talazoparib soft gel capsule formulation under fasting condition) will be Test treatment.

To assess food effect, natural log transformed AUC₂₄ and Cₘₐₓ after multiple dosing on the last day of treatment B and C will be analyzed using a mixed effect model with sequence and treatment as fixed effects and participant within sequence as a random effect. Estimates of the adjusted mean differences (Test-Reference) and corresponding 90% confidence intervals will be obtained from the model. The adjusted mean differences and 90% confidence intervals for the differences will be exponentiated to provide estimates of the ratio of adjusted geometric means (Test/Reference) and 90% confidence intervals for the ratios. Treatment B (the proposed talazoparib soft gel capsule formulation under fasting condition) will be the Reference treatment while Treatment C (the proposed talazoparib soft gel capsule formulation given with high fat/high-calorie meal) will be the Test treatment.

## Claims

1. A pharmaceutical soft gelatin capsule dosage form comprising a soft gelatin shell and a fill, wherein:
(a) the soft gelatin shell comprises acid bone gelatin and at least one plasticizer; and
(b) the fill comprises an anti-oxidant, at least one solvent, and talazoparib or a pharmaceutically acceptable salt thereof.

2. The pharmaceutical soft gelatin capsule dosage form of claim 1, wherein the at least one plasticizer is a) glycerol; b) sorbitol; or c) glycerol and sorbitol.

3. The pharmaceutical soft gelatin capsule dosage form of claim 1 or 2,
wherein the anti-oxidant is tocopherol.

4. The pharmaceutical soft gelatin capsule dosage form of any one of claims 1-3, wherein the at least one solvent is a) polyethylene glycol; b) glycerol; or c) polyethylene glycol and glycerol.

5. A pharmaceutical soft gelatin capsule dosage form of claim 1 comprising a soft gelatin shell and a fill,
wherein the soft gelatin shell comprises:
60 percent to 61 percent by weight of acid bone gelatin,
15 percent to 16 percent by weight of glycerol, and
23 percent of sorbitol; and
wherein, the fill comprises:
0.3 percent by weight of tocopherol,
95 percent by weight of polyethylene glycol,
4 percent by weight of glycerol, and
talazoparib, or a pharmaceutically acceptable salt thereof, in 0.15 percent to 0.3 percent by weight free base equivalent.

6. A pharmaceutical soft gelatin capsule dosage form of claim 1 comprising a soft gelatin shell and a fill,
wherein the soft gelatin shell comprises:
46.270 mg/capsule ± 10% of acid bone gelatin,
11.790 mg/capsule ± 10% of glycerol, and
17.57 mg/capsule ± 10% of sorbitol; and
wherein, the fill comprises:
0.300 mg/capsule ± 10% of tocopherol,
95.555 mg/capsule ± 10% of polyethylene glycol,
4.000 mg/capsule ± 10% of glycerol, and
0.1 mg ± 10% of talazoparib free base, or an equivalent amount of a pharmaceutically acceptable salt thereof.

7. A pharmaceutical soft gelatin capsule dosage form of claim 1 comprising a soft gelatin shell and a fill,
wherein the soft gelatin shell comprises:
61.097 mg/capsule ± 10% of acid bone gelatin,
16.610 mg/capsule ± 10% of glycerol, and
23.190 mg/capsule ± 10% of sorbitol; and
wherein, the fill comprises:
0.375 mg/capsule ± 10% of tocopherol,
119.262 mg/capsule ± 10% of polyethylene glycol,
5.000 mg/capsule ± 10% of glycerol, and
0.25 mg ± 10% of talazoparib free base, or an equivalent amount of a pharmaceutically acceptable salt thereof.

8. A pharmaceutical soft gelatin capsule dosage form of claim 1 comprising a soft gelatin shell and a fill,
wherein the soft gelatin shell comprises:
78.130 mg/capsule ± 10% of acid bone gelatin,
20.250 mg/capsule ± 10% of glycerol, and
29.66 mg/capsule ± 10% of sorbitol; and
wherein, the fill comprises:
0.509 mg/capsule ± 10% of tocopherol,
166.966 mg/capsule ± 10% of polyethylene glycol,
7.000 mg/capsule ± 10% of glycerol, and
0.35 mg ± 10% of talazoparib free base, or an equivalent amount of a pharmaceutically acceptable salt thereof.

9. A pharmaceutical soft gelatin capsule dosage form of claim 1 comprising a soft gelatin shell and a fill,
wherein the soft gelatin shell comprises:
104.930 mg/capsule ± 10% of acid bone gelatin,
26.640 mg/capsule ± 10% of glycerol, and
39.830 mg/capsule ± 10% of sorbitol; and
wherein, the fill comprises:
00.750 mg/capsule ± 10% of tocopherol,
238.523 mg/capsule ± 10% of polyethylene glycol,
10.000 mg/capsule ± 10% of glycerol, and
0.5 mg ± 10% of talazoparib free base, or an equivalent amount of a pharmaceutically acceptable salt thereof.

10. A pharmaceutical soft gelatin capsule dosage form of claim 1 comprising a soft gelatin shell and a fill,
wherein the soft gelatin shell comprises:
141.260 mg/capsule ± 10% of acid bone gelatin,
36.000 mg/capsule ± 10% of glycerol, and
53.620 mg/capsule ± 10% of sorbitol; and
wherein, the fill comprises:
1.125 mg/capsule ± 10% of tocopherol,
357.784 mg/capsule ± 10% of polyethylene glycol,
15.000 mg/capsule ± 10% of glycerol, and
0.75 mg ± 10% of talazoparib free base, or an equivalent amount of a pharmaceutically acceptable salt thereof.

11. A pharmaceutical soft gelatin capsule dosage form of claim 1 comprising a soft gelatin shell and a fill,
wherein the soft gelatin shell comprises:
177.790 mg/capsule ± 10% of acid bone gelatin,
45.310 mg/capsule ± 10% of glycerol, and
67.490 mg/capsule± 10% of sorbitol; and
wherein, the fill comprises;
1.453 mg/capsule ± 10% of tocopherol,
477.047 mg/capsule ± 10% of polyethylene glycol,
20.000 mg/capsule ± 10% of glycerol, and
1 mg ± 10% of talazoparib free base, or an equivalent amount of a pharmaceutically acceptable salt thereof.

12. The pharmaceutical soft gelatin capsule dosage form of claim 2 or 5-11, wherein the sorbitol is anhydrized liquid sorbitol NF.

13. The pharmaceutical soft gelatin capsule dosage form of claim 2 or 5-12, wherein the tocopherol is all-rac-alpha-tocopherol.

14. The pharmaceutical soft gelatin capsule dosage form of any one of claims 1-13, wherein the dosage form comprises talazoparib, or a pharmaceutically acceptable salt thereof, in an amount equivalent to 0.1 mg ± 10% talazoparib free base, in an amount equivalent to 0.25 mg ± 10% talazoparib free base, in an amount equivalent to 0.35 mg ± 10% talazoparib free base, in an amount equivalent to 0.5 mg ± 10% talazoparib free base, in an amount equivalent to 0.75 mg ± 10% talazoparib free base, or in an amount equivalent to 1 mg ± 10% talazoparib free base.

15. The pharmaceutical soft gelatin capsule dosage form of any one of claims 1-14, wherein the talazoparib, or a pharmaceutically acceptable salt thereof, is talazoparib tosylate.

16. The pharmaceutical soft gelatin capsule dosage form of any one of the preceding claims, wherein the dosage form is for oral administration.

17. A pharmaceutical soft gelatin capsule dosage form of any one of claims 1-16 for use in the treatment of cancer.

## Patentansprüche

1. Pharmazeutische Weichgelatinekapsel-Dosierungsform, umfassend eine Weichgelatinehülle und eine Füllung, wobei:
(a) die Weichgelatinehülle saure Knochengelatine und mindestens einen Weichmacher umfasst; und
(b) die Füllung ein Antioxidans, mindestens ein Lösungsmittel und Talazoparib oder ein pharmazeutisch verträgliches Salz davon umfasst.

2. Pharmazeutische Weichgelatinekapsel-Dosierungsform gemäß Anspruch 1, wobei der mindestens eine Weichmacher a) Glycerin; b) Sorbit; oder c) Glycerin und Sorbit ist.

3. Pharmazeutische Weichgelatinekapsel-Dosierungsform gemäß Anspruch 1 oder 2, wobei das Antioxidans Tocopherol ist.

4. Pharmazeutische Weichgelatinekapsel-Dosierungsform gemäß einem der Ansprüche 1 bis 3, wobei das mindestens eine Lösungsmittel a) Polyethylenglykol; b) Glycerin; oder c) Polyethylenglykol und Glycerin ist.

5. Pharmazeutische Weichgelatinekapsel-Dosierungsform gemäß Anspruch 1, umfassend eine Weichgelatinehülle und eine Füllung,
wobei die Weichgelatinehülle umfasst:
60 bis 61 Gewichtsprozent saure Knochengelatine,
15 bis 16 Gewichtsprozent Glycerin,
23 Prozent Sorbit; und
wobei die Füllung umfasst:
0,3 Gewichtsprozent Tocopherol,
95 Gewichtsprozent Polyethylenglykol,
4 Gewichtsprozent Glycerin und
Talazoparib oder ein pharmazeutisch verträgliches Salz davon in einer Menge von 0,15 bis 0,3 Gewichtsprozent als freie Base.

6. Pharmazeutische Weichgelatinekapsel-Dosierungsform gemäß Anspruch 1, umfassend eine Weichgelatinehülle und eine Füllung,
wobei die Weichgelatinehülle umfasst:
46,270 mg/Kapsel ± 10 % saurer Knochengelatine,
11,790 mg/Kapsel ± 10 % Glycerin und
17,57 mg/Kapsel ± 10 % Sorbit; und
wobei die Füllung umfasst:
0,300 mg/Kapsel ± 10 % Tocopherol,
95,555 mg/Kapsel ± 10 % Polyethylenglykol,
4,000 mg/Kapsel ± 10 % Glycerin und
0,1 mg ± 10 % freie Base von Talazoparib oder eine äquivalente Menge eines pharmazeutisch verträglichen Salzes davon.

7. Pharmazeutische -Weichgelatinekapsel-Dosierungsform gemäß Anspruch 1, umfassend eine Weichgelatinehülle und eine Füllung,
wobei die Weichgelatinehülle umfasst:
61,097 mg/Kapsel ± 10 % saure Knochengelatine,
16,610 mg/Kapsel ± 10 % Glycerin und
23,190 mg/Kapsel ± 10 % Sorbit; und
wobei die Füllung umfasst:
0,375 mg/Kapsel ± 10 % Tocopherol,
119,262 mg/Kapsel ± 10 % Polyethylenglykol,
5,000 mg/Kapsel ± 10 % Glycerin, und
0,25 mg ± 10 % freie Base von Talazoparib oder eine äquivalente Menge eines pharmazeutisch verträglichen Salzes davon.

8. Pharmazeutische Weichgelatinekapsel-Dosierungsform gemäß Anspruch 1, umfassend eine Weichgelatinehülle und eine Füllung,
wobei die Weichgelatinehülle umfasst:
78,130 mg/Kapsel ± 10 % saure Knochengelatine,
20,250 mg/Kapsel ± 10 % Glycerin und
29,66 mg/Kapsel ± 10 % Sorbit; und
wobei die Füllung umfasst:
0,509 mg/Kapsel ± 10 % Tocopherol,
166,966 mg/Kapsel ± 10 % Polyethylenglykol,
7,000 mg/Kapsel ± 10 % Glycerin und
0,35 mg ± 10 % freie Base von Talazoparib oder eine äquivalente Menge eines pharmazeutisch verträglichen Salzes davon.

9. Pharmazeutische Weichgelatinekapsel-Dosierungsform gemäß Anspruch 1, umfassend eine Weichgelatinehülle und eine Füllung,
wobei die Weichgelatinehülle umfasst:
104,930 mg/Kapsel ± 10 % saure Knochengelatine,
26,640 mg/Kapsel ± 10 % Glycerin, und
39,830 mg/Kapsel ± 10 % Sorbit; und
wobei die Füllung umfasst:
0,750 mg/Kapsel ± 10 % Tocopherol,
238,523 mg/Kapsel ± 10 % Polyethylenglykol,
10,000 mg/Kapsel ± 10 % Glycerin, und
0,5 mg ± 10 % freie Base von Talazoparib oder eine äquivalente Menge eines pharmazeutisch verträglichen Salzes davon.

10. Pharmazeutische Weichgelatinekapsel-Dosierungsform gemäß Anspruch 1, umfassend eine Weichgelatinehülle und eine Füllung,
wobei die Weichgelatinehülle umfasst:
141,260 mg/Kapsel ± 10 % saure Knochengelatine,
36,000 mg/Kapsel ± 10 % Glycerin, und
53,620 mg/Kapsel ± 10 % Sorbit; und
wobei die Füllung umfasst:
1,125 mg/Kapsel ± 10 % Tocopherol,
357,784 mg/Kapsel ± 10 % Polyethylenglykol,
15,000 mg/Kapsel ± 10 % Glycerin und
0,75 mg ± 10 % freie Base von Talazoparib oder eine äquivalente Menge eines pharmazeutisch verträglichen Salzes davon.

11. Pharmazeutische Weichgelatinekapsel-Dosierungsform gemäß Anspruch 1, umfassend eine Weichgelatinehülle und eine Füllung,
wobei die Weichgelatinehülle umfasst:
177,790 mg/Kapsel ± 10 % saure Knochengelatine,
45,310 mg/Kapsel ± 10 % Glycerin, und
67,490 mg/Kapsel ± 10 % Sorbit; und
wobei die Füllung umfasst:
1,453 mg/Kapsel ± 10 % Tocopherol,
477,047 mg/Kapsel ± 10 % Polyethylenglykol,
20,000 mg/Kapsel ± 10 % Glycerin, und
1 mg ± 10 % freie Base von Talazoparib oder eine äquivalente Menge eines pharmazeutisch verträglichen Salzes davon.

12. Pharmazeutische Weichgelatinekapsel-Dosierungsform gemäß Anspruch 2 oder 5-11, wobei das Sorbit ein Sorbit-Anhydrid-Flüssigsorbit NF ist.

13. Pharmazeutische Weichgelatinekapsel-Dosierungsform gemäß Anspruch 2 oder 5-12, wobei das Tocopherol All-rac-alpha-Tocopherol ist.

14. Pharmazeutische Weichgelatinekapsel-Dosierungsform gemäß einem der Ansprüche 1-13, wobei die Dosierungsform Talazoparib oder ein pharmazeutisch verträgliches Salz davon in einer Menge umfasst, die 0,1 mg ± 10 % freier Base von Talazoparib entspricht, in einer Menge, die 0,25 mg ± 10 % freier Base von Talazoparib entspricht, in einer Menge, die 0,35 mg ± 10 % freier Base von Talazoparib entspricht, in einer Menge, die 0,5 mg ± 10 % freier Base von Talazoparib entspricht, in einer Menge, die 0,75 mg ± 10 % freier Base von Talazoparib entspricht, oder in einer Menge, die 1 mg ± 10 % freier Base von Talazoparib entspricht.

15. Pharmazeutische Weichgelatinekapsel-Dosierungsform gemäß einem der Ansprüche 1 bis 14, wobei das Talazoparib oder ein pharmazeutisch verträgliches Salz davon Talazoparibtosylat ist.

16. Pharmazeutische Weichgelatinekapsel-Dosierungsform gemäß einem der vorherigen Ansprüche, wobei die Dosierungsform zur oralen Verabreichung bestimmt ist.

17. Pharmazeutische Weichgelatinekapsel-Dosierungsform gemäß einem der Ansprüche 1 bis 16 zur Verwendung bei der Behandlung von Krebs.

## Revendications

1. Forme posologique pharmaceutique de type capsule en gélatine molle comprenant une coque de gélatine molle et une charge, dans laquelle :
(a) la coque de gélatine souple comprend de la gélatine osseuse acide et au moins un plastifiant ; et
(b) la charge comprend un anti-oxydant, au moins un solvant, et du talazoparib ou un sel pharmaceutiquement acceptable de celui-ci.

2. Forme posologique pharmaceutique de type capsule en gélatine molle selon la revendication 1, dans laquelle l'au moins un plastifiant est a) le glycérol ; b) le sorbitol ; ou c) le glycérol et le sorbitol.

3. Forme posologique pharmaceutique de type capsule en gélatine molle selon la revendication 1 ou 2, dans laquelle l'anti-oxydant est du tocophérol.

4. Forme posologique pharmaceutique de type capsule en gélatine molle selon l'une des revendications 1 à 3, dans laquelle l'au moins un solvant est a) le polyéthylène glycol ; b) le glycérol ; ou c) le polyéthylène glycol et le glycérol.

5. Forme posologique pharmaceutique de type capsule en gélatine molle selon la revendication 1 comprenant une coque de gélatine molle et une charge,
dans laquelle la coque de gélatine molle comprend :
60 pour cent à 61 pour cent en poids de gélatine osseuse acide,
15 pour cent à 16 pour cent en poids de glycérol, et
23 pour cent de sorbitol ; et
dans laquelle la charge comprend :
0,3 pour cent en poids de tocophérol,
95 pour cent en poids de polyéthylène glycol,
4 pour cent en poids de glycérol, et
du talazoparib, ou un sel pharmaceutiquement acceptable de celui-ci, selon un équivalent base libre de 0,15 pour cent à 0,3 pour cent en poids.

6. Forme posologique pharmaceutique de type capsule en gélatine molle selon la revendication 1 comprenant une coque de gélatine molle et une charge,
dans laquelle la coque de gélatine molle comprend :
46,270 mg/capsule ± 10 % de gélatine osseuse acide,
11,790 mg/capsule ± 10 % de glycérol, et
17,57 mg/capsule ± 10 % de sorbitol ; et
dans laquelle la charge comprend :
0,300 mg/capsule ± 10 % de tocophérol,
95,555 mg/capsule ± 10 % de polyéthylène glycol,
4,000 mg/capsule ± 10 % de glycérol, et
0,1 mg ± 10 % de base libre de talazoparib, ou une quantité équivalente d'un sel pharmaceutiquement acceptable de celui-ci.

7. Forme posologique pharmaceutique de type capsule en gélatine molle selon la revendication 1 comprenant une coque de gélatine molle et une charge,
dans laquelle la coque de gélatine molle comprend :
61,097 mg/capsule ± 10 % de gélatine osseuse acide,
16,610 mg/capsule ± 10 % de glycérol, et
23,190 mg/capsule ± 10 % de sorbitol ; et
dans laquelle la charge comprend :
0,375 mg/capsule ± 10 % de tocophérol,
119,262 mg/capsule ± 10 % de polyéthylène glycol,
5,000 mg/capsule ± 10 % de glycérol, et
0,25 mg ± 10 % de base libre de talazoparib, ou une quantité équivalente d'un sel pharmaceutiquement acceptable de celui-ci.

8. Forme posologique pharmaceutique de type capsule en gélatine molle selon la revendication 1 comprenant une coque de gélatine molle et une charge,
dans laquelle la coque de gélatine molle comprend :
78,130 mg/capsule ± 10 % de gélatine osseuse acide,
20,250 mg/capsule ± 10 % de glycérol, et
29,66 mg/capsule ± 10 % de sorbitol ; et
dans laquelle la charge comprend :
0,509 mg/capsule ± 10 % de tocophérol,
166,966 mg/capsule ± 10 % de polyéthylène glycol,
7,000 mg/capsule ± 10 % de glycérol, et
0,35 mg ± 10 % de base libre de talazoparib, ou une quantité équivalente d'un sel pharmaceutiquement acceptable de celui-ci.

9. Forme posologique pharmaceutique de type capsule en gélatine molle selon la revendication 1 comprenant une coque de gélatine molle et une charge,
dans laquelle la coque de gélatine molle comprend :
104,930 mg/capsule ± 10 % de gélatine osseuse acide,
26,640 mg/capsule ± 10 % de glycérol, et
39,830 mg/capsule ± 10 % de sorbitol ; et
dans laquelle la charge comprend :
00,750 mg/capsule ± 10 % de tocophérol,
238,523 mg/capsule ± 10 % de polyéthylène glycol,
10,000 mg/capsule ± 10 % de glycérol, et
0,5 mg ± 10 % de base libre de talazoparib, ou une quantité équivalente d'un sel pharmaceutiquement acceptable de celui-ci.

10. Forme posologique pharmaceutique de type capsule en gélatine molle selon la revendication 1 comprenant une coque de gélatine molle et une charge,
dans laquelle la coque de gélatine molle comprend :
141,260 mg/capsule ± 10 % de gélatine osseuse acide,
36,000 mg/capsule ± 10 % de glycérol, et
53,620 mg/capsule ± 10 % de sorbitol ; et
dans laquelle la charge comprend :
1,125 mg/capsule ± 10 % de tocophérol,
357,784 mg/capsule ± 10 % de polyéthylène glycol,
15,000 mg/capsule ± 10 % de glycérol, et
0,75 mg ± 10 % de base libre de talazoparib, ou une quantité équivalente d'un sel pharmaceutiquement acceptable de celui-ci.

11. Forme posologique pharmaceutique de type capsule en gélatine molle selon la revendication 1 comprenant une coque de gélatine molle et une charge,
dans laquelle la coque de gélatine molle comprend :
177,790 mg/capsule ± 10 % de gélatine osseuse acide,
45,310 mg/capsule ± 10 % de glycérol, et
67,490 mg/capsule ± 10 % de sorbitol ; et
dans laquelle la charge comprend ;
1,453 mg/capsule ± 10 % de tocophérol,
477,047 mg/capsule ± 10 % de polyéthylène glycol,
20,000 mg/capsule ± 10 % de glycérol, et
1 mg ± 10 % de base libre de talazoparib, ou une quantité équivalente d'un sel pharmaceutiquement acceptable de celui-ci.

12. Forme posologique pharmaceutique de type capsule en gélatine molle selon la revendication 2 ou les revendications 5 à 11, dans laquelle le sorbitol est du sorbitol liquide anhydre NF.

13. Forme posologique pharmaceutique de type capsule en gélatine molle selon la revendication 2 ou les revendications 5 à 12, dans laquelle le tocophérol est du all-rac-alpha-tocophérol.

14. Forme posologique pharmaceutique de type capsule en gélatine molle selon l'une des revendications 1 à 13, dans laquelle la forme posologique comprend du talazoparib, ou un sel pharmaceutiquement acceptable de celui-ci, selon une quantité équivalente à 0,1 mg ± 10 % de base libre de talazoparib, selon une quantité équivalente à 0,25 mg ± 10 % de base libre de talazoparib, selon une quantité équivalente à 0,35 mg ± 10 % de base libre de talazoparib, selon une quantité équivalente à 0,5 mg ± 10 % de base libre de talazoparib, selon une quantité équivalente à 0,75 mg ± 10 % de base libre de talazoparib, ou selon une quantité équivalente à 1 mg ± 10 % de base libre de talazoparib.

15. Forme posologique pharmaceutique de type capsule en gélatine molle selon l'une des revendications 1 à 14, dans laquelle le talazoparib, ou un sel pharmaceutiquement acceptable de celui-ci, est du tosylate de talazoparib.

16. Forme posologique pharmaceutique de type capsule en gélatine molle selon l'une des revendications précédentes, dans laquelle la forme posologique est destinée à une administration orale.

17. Forme posologique pharmaceutique de type capsule en gélatine molle selon l'une des revendications 1 à 16 pour une utilisation dans le traitement de cancer.
